(19) Europäisches Patentamt / European Patent Office / Office européen des brevets

(11) **EP 3 743 031 B1**

(12) **FASCICULE DE BREVET EUROPEEN**

(45) Date de publication et mention
de la délivrance du brevet:
**18.06.2025 Bulletin 2025/25**

(21) Numéro de dépôt: **19710019.1**

(22) Date de dépôt: **25.01.2019**

(51) Classification Internationale des Brevets (IPC):
***A61F 13/62*** *(2006.01)*

(52) Classification Coopérative des Brevets (CPC):
**A61F 13/62; A61F 13/625**

(86) Numéro de dépôt international:
**PCT/FR2019/050163**

(87) Numéro de publication internationale:
**WO 2019/145647 (01.08.2019 Gazette 2019/31)**

(54) **DISPOSITIF DE RETENUE ET RUBAN POUR DISPOSITIF DE RETENUE**

HALTEVORRICHTUNG UND STREIFEN FÜR EINE HALTEVORRICHTUNG

RETAINING DEVICE, AND STRIP FOR A RETAINING DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **26.01.2018 FR 1850645**

(43) Date de publication de la demande:
**02.12.2020 Bulletin 2020/49**

(73) Titulaire: **Aplix**
**44850 Le Cellier (FR)**

(72) Inventeur: **BOSSER, Damien**
**44850 LE CELLIER (FR)**

(74) Mandataire: **Cabinet Beau de Loménie**
**158, rue de l'Université**
**75340 Paris Cedex 07 (FR)**

(56) Documents cités:
**US-A1- 2014 338 159     US-A1- 2014 358 107
US-A1- 2016 128 877**

• **ANONYMOUS: "Paillasson d'extérieur en pin
lazuré et poils en nylon", 3 November 2016
(2016-11-03), XP055513565, Retrieved from the
Internet <URL:https://www.landmade.fr/
boutique/pelles-et-paillassons/
41-paillasson-40x60.html> [retrieved on
20181009]**

**Description**

Domaine technologique

Le présent exposé concerne un dispositif de retenue comprenant des éléments en saillies, tels que des tiges et/ou des préformes et/ou des crochets, par exemple destiné à coopérer avec une contrepartie à crochets et/ou à boucles.

Arrière-plan technologique

Notamment dans le domaine de l'hygiène, des dispositifs de retenue, par exemple à crochets, sont utilisés et coopèrent avec une contrepartie à boucles formant une zone d'application du dispositif de retenue. Des exemples de dispositifs de retenue sont décrits dans les documents US2014/0358107 et US2016/128877.

Lorsqu'un utilisateur positionne le dispositif de retenue sur cette zone d'application, l'utilisateur peut douter qu'il a positionné correctement le dispositif de retenue sur la zone d'application. Cette zone d'application est communément appelée dans le domaine de l'hygiène « bande confort » ou identifiée par l'expression en anglais « landing zone ». Le fait que l'utilisateur soit certain d'avoir positionné correctement le dispositif de retenue sur la zone d'application va influer sur la perception que l'utilisateur a du fait que le dispositif de retenue est bien maintenu sur la zone d'application.

L'utilisateur peut être également amené à repositionner le dispositif de retenue sur la zone d'application, par exemple afin de mieux ajuster l'article et/ou afin de retirer l'article.

La force de pelage est typiquement la force que l'utilisateur va exercer sur le dispositif de retenue pour séparer le dispositif de retenue de la zone d'application. La facilité avec laquelle l'utilisateur peut séparer le dispositif de retenue de la zone d'application va influer sur la perception que l'utilisateur a du fait que le dispositif de retenue est bien maintenu sur la zone d'application. Dans certains cas extrêmes, une force de pelage faible peut provoquer un détachement inopiné du dispositif de retenue et de la zone d'application.

Il existe donc un besoin d'améliorer la sensation/qualité réelle et/ou perçue par utilisateur lorsque qu'il utilise le dispositif de retenue en fermeture et/ou en ouverture.

Présentation

Le présent exposé vise à remédier au moins en partie à ces inconvénients en proposant un dispositif de retenue simple à utiliser, visuel, cognitif, intuitif pour l'utilisateur.

A cet effet, le présent exposé concerne un dispositif de retenue destiné à coopérer avec une contrepartie à crochets et/ou à boucles comprenant :

- une base continue présentant une face supérieure et une face inférieure, la base ayant une épaisseur, mesurée perpendiculairement à la face supérieure de la base ; et
- une pluralité d'éléments de retenue s'étendant de la face supérieure de la base, les éléments de retenue étant des tiges et/ou des préformes et/ou des crochets, chaque élément de retenue comprenant une tige, une hauteur des éléments de retenue, mesurée perpendiculairement à la face supérieure de la base est comprise entre 3 et 10 fois l'épaisseur de la base ;

la base comportant au moins une zone continue selon une direction perpendiculaire à une direction de pelage, la zone étant dépourvue d'éléments de retenue de sorte que la pluralité d'éléments de retenue forme au moins deux motifs et dans lequel le motif est répétitif selon la direction de pelage, la base comportant une alternance de zones dépourvues d'éléments de retenue et de motifs, la force de pelage mesurée selon la méthode « Pelage 180° » présentant au moins deux pics et au moins une vallée comprise entre les deux pics, des valeurs maximales des pics étant croissantes avec la course d'ouverture et la au moins une vallée présentant une valeur minimale inférieure ou égale à 85% d'une valeur maximale de la force de pelage, de préférence inférieure ou égale à 70%, encore plus de préférence inférieure ou égale à 60%, encore plus préférentiellement inférieure ou égale à 50%, encore plus préférentiellement inférieure ou égale à 40%, une distance minimale entre deux éléments de retenue étant comprise entre 0,1 mm et 10 mm, et le motif présentant une densité d'éléments de retenue supérieure ou égale à 20 éléments de retenue par $cm^2$.

En variante, dans un espace de couleur CIE L*a*b*, une différence de couleur $\Delta E^*$ entre la au moins une zone dépourvues d'éléments de retenue et au moins une partie du motif formé par la pluralité d'éléments de retenue est supérieure ou égale à 1,0, de préférence supérieure ou égale à 1,5, encore plus de préférence supérieure ou égale à 3,0, encore plus de préférence supérieure ou égale à 4,5.

Par motif, on comprend que la répartition des éléments de retenue n'est pas uniforme sur toute la base. Ainsi, bien que les éléments de retenue puissent être espacés de manière uniforme sur la base pour former le motif, certaines zones de la base sont dépourvues d'éléments de retenue et permettent de délimiter le motif.

Le motif comprend une surface de motif correspondant à la surface couverte par des cercles de rayon correspondant au pas moyen et dont le centre de chaque cercle est positionné respectivement, en vue du dessus, sur le centre des éléments de retenue et la circonférence de chaque cercle passe par le centre d'au moins un élément de retenue adjacent. Le pas moyen peut correspondre à la distance séparant deux éléments de retenue adjacents. La au moins une zone dépourvue d'éléments de retenue est la surface non couverte par la surface de motif.

La différence de couleur ΔE* entre les zones dépourvues d'éléments de retenue et au moins une partie du motif formé par la pluralité d'éléments de retenue est supérieure ou égale à 1,0, l'utilisateur peut identifier visuellement le motif formé par la pluralité d'élément de retenue et donc être certain qu'il positionne bien le motif sur une zone d'application, telle une contrepartie à boucles par exemple. Cela permet donc d'améliorer la sensation/qualité réelle et/ou perçue par utilisateur lorsque qu'il utilise le dispositif de retenue en fermeture et/ou en ouverture.

L'espace de couleur CIE L*a*b* ou espace chromatique CIE L*a*b*, généralement nommé CIELAB, est l'espace utilisé de manière la plus répandue et est émis par la Commission Internationale de l'Eclairage (CIE) afin de caractériser des couleurs de surface. Cet espace décrit toutes les couleurs visibles pour l'œil humain et a été créée afin de servir de référence. Dans cet espace, la clarté L* varie de 0 (= noir) à 100 (= blanc), le paramètre a* représente la valeur sur un axe variant de vert à rouge et le paramètre b* représente la valeur sur un axe variant de bleu à jaune. La différence de couleur ΔE* entre les zones dépourvues d'éléments de retenue et au moins une partie du motif formé par la pluralité d'éléments de retenue est calculée selon l'équation (1) :

$$(1)\, \Delta E^* = \sqrt{\Delta L^{*2} + \Delta a^{*2} + \Delta b^{*2}}$$

Les valeurs de L*, a* et b* sont mesurées par exemple avec un spectrocolorimètre en lumière naturelle (sous la référence D65/10°) de modèle RM200QC de x-rite pantone sur un support blanc. Ce spectromètre est en particulier bien adapté pour mesurer des surfaces supérieures ou égales à 4 mm$^2$.

Dans certains modes de réalisation, il existe plusieurs zones pourvues d'éléments de retenue, chaque zone pourvue d'éléments de retenue présentant, dans l'espace de couleur CIE L*a*b*, une différence de couleur ΔE* entre la au moins une zone dépourvue d'éléments de retenue et au moins une partie du motif formé par la pluralité d'éléments de retenue, la différence de couleur ΔE* étant supérieure ou égale à 1,0, de préférence supérieure ou égale à 1,5, encore plus de préférence supérieure ou égale à 3,0 encore plus de préférence supérieure ou égale à 4,5.

Dans certains modes de réalisation, il existe plusieurs zones pourvues d'éléments de retenue, chaque zone pourvue d'éléments de retenue présentant, dans l'espace de couleur CIE L*a*b*, une différence de couleur ΔE* entre une première zone pourvue d'éléments de retenue et une deuxième zone pourvue d'éléments de retenue, la différence de couleur ΔE* étant supérieure ou égale à 1,0, de préférence supérieure ou égale à 1,5, encore plus de préférence supérieure ou égale à 3,0 encore plus de préférence supérieure ou égale à 4,5.

Dans certains modes de réalisation, l'élément de retenue comprend la tige surmontée d'une tête.

La tige comprend une extrémité inférieure reliée à la base, et une extrémité supérieure opposée à l'extrémité inférieure et la tête surmonte l'extrémité supérieure de la tige.

Ce type d'élément de retenue peut par exemple être une préforme et/ou un crochet.

Dans certains modes de réalisation, une surface des zones dépourvues d'éléments de retenue est supérieure ou égale à 5% d'une surface totale de la base, de préférence est supérieure ou égale à 10%, encore plus de préférence est supérieure ou égale à 15%.

Généralement, les éléments de retenue ne sont pas présents sur les bords de la base. Ainsi, pour des dispositifs de retenue ne comprenant pas de motif, c'est-à-dire des dispositifs de retenue dans lesquels les éléments de retenue sont répartis uniformément sur toute la base, il peut exister une bande, le long des bords de la base dépourvus d'éléments de retenue. Toutefois, ces bandes ne présentent pas une surface suffisante pour permettre une identification par l'utilisateur d'un motif du fait de leur forme droite. L'utilisateur n'identifie donc pas la présence de zones dépourvues d'éléments de retenue comme des zones délimitant un motif. On comprend que les zones étroites formant généralement une bande en bordure de dispositif de retenue ne sont pas bordées de chaque côté par des dispositifs de retenue.

Dans certains modes de réalisation, le motif est un motif unique.

On entend par motif unique un motif isolé dans une zone de la base mesurant 25,4 mm ou 30,0 mm ou 35,0 mm dans la direction MD et 13,0 mm ou 25,0 mm ou 25,4 mm dans la direction CD.

On entend par direction MD la direction de déplacement de la base dans la machine lors de la fabrication du dispositif de retenue, conformément au sigle anglais pour « Machine Direction », et par direction CD, conformément au sigle anglais pour « Cross Direction », la direction perpendiculaire à la direction MD.

Ce motif peut par exemple représenter un motif reconnaissable par l'utilisateur, par exemple un signe distinctif ou une marque.

Dans certains modes de réalisation, le motif est un motif répétitif.

Le motif peut par exemple être de dimension plus réduite, en comparaison à un motif unique, mais être répété plusieurs fois.

Dans certains modes de réalisation, le motif répété peut être un motif complexe.

On entend par motif complexe un motif comprenant plusieurs entités qui peuvent être différentes les unes des autres, ce motif complexe étant lui-même répété au moins une fois.

Dans certains modes de réalisation, le motif présente un contour fermé.

On entend par contour fermé une courbe dont les deux extrémités sont confondues.

Dans certains modes de réalisation, le motif est agencé de sorte que lorsque le dispositif de retenue est sollicité en ouverture, une force exercée sur le dispositif de retenue est décomposée entre au moins une première composante en force de pelage et une deuxième composante en force de cisaillement.

Dans certains modes de réalisation, dans un espace de couleur CIE XYZ, la différence d'opacité entre la au moins une zone dépourvue d'éléments de retenue et au moins une partie du motif formé par la pluralité d'éléments de retenue est supérieure ou égale à 1, de préférence supérieure ou égale à 2, encore plus de préférence supérieure ou égale à 3.

L'espace de couleur CIE XYZ est un espace de couleur défini par la Commission Internationale de l'Eclairage. La composante Y de l'espace CIE XYZ correspond à la luminance, c'est-à-dire à la luminosité d'une surface. Selon la norme ASTM D2805, l'opacité exprimée en pourcent est calculée selon l'équation (2) :

$$(2) \ Opacité \ (\%) = \frac{Y_{fond \ noir}}{Y_{fond \ blanc}} * 100$$

où $Y_{fond \ noir}$ est la mesure de Y réalisée sur un support noir et $Y_{fond \ blanc}$ est la mesure de Y réalisée sur un support blanc.

Les valeurs de $Y_{fond \ noir}$ et $Y_{fond \ blanc}$ sont mesurées par exemple avec un spectrocolorimètre en lumière naturelle (sous la référence D65/10°) de modèle RM200QC de x-rite pantone sur un support blanc et sur un support noir.

La différence d'opacité entre les zones dépourvues d'éléments de retenue et au moins une partie du motif formé par la pluralité d'éléments de retenue est égale à la valeur absolue de la différence des valeurs d'opacité obtenues pour la zone dépourvue d'éléments de retenue et pour au moins une partie du motif formé par la pluralité d'éléments de retenue.

Dans certains modes de réalisation, il existe plusieurs zones pourvues d'éléments de retenue, chaque zone pourvue d'éléments de retenue présentant, dans l'espace de couleur CIE XYZ, une différence d'opacité entre la au moins une zone dépourvue d'éléments de retenue et au moins une partie du motif formé par la pluralité d'éléments de retenue, la différence d'opacité étant supérieure ou égale à 1, de préférence supérieure ou égale à 2, encore plus de préférence supérieure ou égale à 3.

Dans certains modes de réalisation, il existe plusieurs zones pourvues d'éléments de retenue, chaque zone pourvue d'éléments de retenue présentant, dans l'espace de couleur CIE XYZ, une différence d'opacité entre une première zone pourvue d'éléments de retenue et une deuxième zone pourvue d'éléments de retenue, la différence d'opacité étant supérieure ou égale à 1, de préférence supérieure ou égale à 2, encore plus de préférence supérieure ou égale à 3.

Dans certains modes de réalisation, la base et la pluralité d'éléments de retenue sont en matériau thermoplastique.

Comme exemple non limitatif de matériau thermoplastique, on peut citer une polyoléfine, le polyéthylène, le LLDPE (Linear Low Density PolyEthylène ou polyéthylène linéaire de faible densité), LDPE (Low Density PolyEthylène ou polyéthylène de faible densité), m-PE (Métallocène PolyEthylène), HDPE (High Density PolyEthylène ou polyéthylène de haute densité), EVA (Éthylène Acétate de Vinyle) et PP (PolyPropylène), comprenant une distribution de poids moléculaire monomodale ou multimodale (par exemple bimodale), en particulier une composition comprenant du LLDPE et d'un plastomère, notamment d'un plastomère base polyéthylène. On pourrait également utiliser du polyamide (PA), de l'acide polyactique (PLA), du polyhydroxyalcanoates (PHA), PVOH, PBS, le polyester, le polychlorure de vinyle (PVC) ou de l'acrylonitrile butadiène styrène (ABS).

Dans certains modes de réalisation, la base et la pluralité d'éléments de retenue sont en matériau thermoplastique comprenant un colorant.

Le colorant peut être par exemple un colorant blanc, par exemple on peut citer la référence 50PP commercialisée par CABOT et qui est chargé à 50% massique de $TiO_2$. Comme autre colorant, on peut également citer la référence UN55206 fabriqué par COLOR SERVICE. Ces exemples sont donnés à titre non limitatif.

Le colorant peut permettre d'augmenter le contraste visuel entre les zones dépourvues d'éléments de retenue et le motif formé par la pluralité d'éléments de retenue.

Dans certains modes de réalisation, la base et la pluralité d'éléments de retenue sont en matériau thermoplastique comprenant au plus 2% massique de colorant, de préférence au plus 1,5% massique, encore plus de préférence au plus 1% massique de colorant.

Dans certains modes de réalisation, une extrémité de chaque élément de retenue la plus éloignée de la base comporte un revêtement coloré.

Ainsi, on peut déposer un revêtement coloré, par exemple une encre ou un colorant, tels que ceux couramment utilisés en flexographie et/ou en tampographie et/ou en rotogravure et/ou en sérigraphie et/ou en héliographie, afin de déposer le revêtement coloré sur l'extrémité de chaque élément de retenue la plus éloignée de la base. L'encre peut être une encre à base solvant ou à base eau ou encore réticulable sous ultraviolets.

Une encre est généralement composée d'un mélange de trois composants : une matière colorante, notamment un pigment ou un colorant ; un véhicule formant la phase fluide de l'encre, par exemple un mélange de polymères, de diluants et/ou de solvant ou encore d'eau ; et des additifs, tels que agents dispersants, anti-mousse, etc., permettant d'optimiser les caractéristiques de l'encre.

Comme mélange de polymères, on peut utiliser des mélanges comprenant par exemple jusqu'à 50% massique de divers acétates, tels que l'acétate d'éthyle, l'acétate de N propyle, acétate d'isopropyle, acétate de N butyle, et leurs mélanges et/ou jusqu'à 10% massique d'alcool.

On peut utiliser des pigments organiques ou minéraux, comme par exemple des colorants diazoïques, des colorants anthraquinone, xanthène, azine et analogues, dioxyde de titane, noir de carbone, oxydes de fer, oxyde de chrome et analogues.

Comme exemple non limitatif d'encre, on peut utiliser une encre commercialisée par Encre ULTRA sous la référence « Type Série 30 000 ALC polyamide » ou une encre commercialisée par la société DOMECK EUROFLEX sous la référence « Type Série EURO-Film PXA ».

On comprend que ce revêtement coloré peut être déposé sur un matériau thermoplastique non coloré ou coloré, et ce afin d'augmenter le contraste entre les zones dépourvues d'éléments de retenue et le motif formé par la pluralité d'éléments de retenue.

Typiquement, un rouleau encreur est enduit d'encre ou de colorant et le dispositif de retenue est entraîné de sorte que seuls les éléments de retenue soient mis en contact avec le rouleau encreur. L'extrémité la plus éloignée de la base de chaque élément de retenue sera ainsi recouvert d'un revêtement coloré permettant d'accentuer la visibilité du motif.

La méthode « Pelage 180° » est une méthode qui permet de mesurer la force de pelage, c'est-à-dire la force pour séparer le dispositif de retenue et la zone d'application. Cette méthode est décrite ci-dessous.

Conditionnement des échantillons - Les échantillons à tester sont conditionnés pendant 2 h (heure) à 23°C+/-2°C avec une humidité relative de 50%+/-5%.

Préparation du dispositif de retenue - Le dispositif de retenue est généralement utilisé dans la direction CD. Le dispositif de retenue se présente généralement sous forme d'un ruban dont la longueur est dans la direction MD. Une partie du ruban selon la direction MD est collée sur un papier de 80 g/cm$^2$ et un rouleau de 2 kg (kilogramme) est appliqué ou passé en rotation sur le dispositif de retenue dans un sens et puis dans l'autre (aller-retour) sur toute la longueur de la partie du ruban. Le papier et le dispositif de retenue sont découpés à l'aide d'une paire de ciseaux en bandelettes de 25,4 mm (millimètre) de largeur dans la direction CD à une vitesse d'environ 700 mm/min (millimètre par minute). Chaque bandelette de papier présente une longueur de 210 mm et le dispositif de retenue est disposé au centre de cette bandelette.

Préparation de la zone d'application - L'échantillon de la zone d'application présente une largeur de 50 mm dans la direction MD et la longueur est au maximum de 200 mm et l'échantillon est coupé en deux selon la longueur.

Assemblage - La bandelette est disposée sur l'échantillon de la zone d'application de sorte que le dispositif de retenue soit centré sur l'échantillon de la zone d'application. Le rouleau de 2 kg (kilogramme) est appliqué ou passé en rotation sur la bandelette dans un sens et puis dans l'autre (aller-retour) sur toute la longueur de la bandelette à une vitesse d'environ 700 mm/min. L'échantillon de la zone d'application est disposé dans une pince d'une potence, le côté coupé étant dans la pince et un poids de 1 kg est suspendu à la partie inférieure de la bandelette pendant 10 s (seconde). Le poids est ensuite retiré. Cette étape permet de s'assurer de l'assemblage correct du dispositif de retenue et de l'échantillon de la zone d'application.

Mesure - L'ensemble est ensuite disposé dans une machine d'essai en traction comprenant une cellule de mesure de 100 N (newton). La bandelette est insérée dans la mâchoire supérieure (mobile). On met la lecture de la cellule de mesure de force à zéro. On insère l'échantillon de la zone d'application dans la mâchoire inférieure (fixe) et on crée une légère tension. La force doit être comprise entre 0,02 N et 0,05 N. Lors de la mise en place, les mâchoires sont écartées l'une de l'autre de 50 mm. L'ensemble est centré entre les deux mâchoires. Le test est réalisé à déplacement constant à une vitesse de 305 mm/min et la course d'essai est de 50 mm. Cette course d'essai est adaptée en fonction de la largeur du dispositif de retenue à tester.

On comprend que la vallée est située entre deux pics successifs. Les courbes de pelage n'étant pas régulières, afin de différencier un pic d'un autre, on considère que l'on a un nouveau pic lorsque la différence de valeur maximale de deux pics est au moins de 10% de la valeur maximale de la force la plus importante mesurée, c'est-à-dire supérieure à 10% de valeur maximale du pic le plus grand. Les pics et les vallées ont une pointe et une base, la base peut présenter une largeur de préférence supérieure à 1 mm, plus particulièrement une largeur supérieure à 2 mm, dans certains cas, une largeur supérieure à 3 mm.

Grâce à la force de pelage qui présente au moins deux pics consécutifs séparés par une vallée, la valeur des pics de la force de pelage allant croissante avec la course d'ouverture, l'utilisateur ressent cette force croissante requise pour séparer le dispositif de retenue de la zone **d'application.** Il perçoit donc que le dispositif de retenue était bien maintenu sur la zone d'application. Par ailleurs, la valeur maximale de la force de pelage est telle que le dispositif de retenue ne peut pas se détacher de la zone d'application de manière non désirée.

On considère généralement qu'une force de pelage, obtenue selon la méthode « Pelage 180° », qui est supérieure ou égale à 1,8 N permet d'éviter un détachement inopiné du dispositif de retenue et de la zone d'application et ce, indépendamment du dispositif de retenue et de la zone d'application.

Dans certains modes de réalisation, le motif est répétitif selon une direction de pelage, la force de pelage mesurée selon la méthode « Pelage 180° » présente au moins trois pics et au moins deux vallées, chaque vallée étant comprise entre deux pics consécutifs, les valeurs maximales des pics étant croissantes avec la course d'ouverture et les vallées présentant une valeur minimale inférieure ou égale à 85% d'une valeur maximale de la force de pelage, de préférence inférieure ou égale à 70%, encore plus de préférence inférieure ou égale à 60%, encore plus préférentiellement inférieure ou égale à 50%, encore plus préférentiellement inférieure ou égale à 40%.

Dans certains modes de réalisation, les zones dépourvues d'éléments de retenue qui sont continues selon une direction perpendiculaire à la direction de pelage présentent une largeur mesurée dans la direction de pelage supérieure ou égale à 2 rangées d'éléments de retenue mesurées dans la direction de pelage, de préférence supérieure ou égale à 3 rangées d'éléments de retenue mesurées dans la direction de pelage.

Bien que ces zones continues dépourvues d'éléments de retenue soient de faible largeur, elles permettent de définir un motif formé par les éléments de retenue du fait qu'elles sont bordées de chaque côté par des éléments de retenue.

Dans certains modes de réalisation, les zones dépourvues d'éléments de retenue qui sont continues selon une direction perpendiculaire à la direction de pelage présentent une largeur mesurée dans la direction de pelage supérieure ou égale à 1% de la largeur de la base mesurée dans la direction de pelage, de préférence supérieure ou égale à 2%, de préférence supérieure ou égale à 4%, encore plus de préférence supérieure ou égale à 5%, encore plus de préférence supérieure ou égale à 10%.

Dans certains modes de réalisation, le dispositif de retenue comprend une bande de tissé ou de non-tissé ou un film thermoplastique ou un film élastique ou un film composite.

Cette bande de tissé ou de non-tissé ou le film thermoplastique ou le film élastique ou le film composite sert de support à la base.

On entend par non-tissé un produit obtenu à l'issue de la formation d'une nappe de fibres et/ou de filaments qui ont été consolidés. La consolidation peut être mécanique, chimique ou thermique et se traduit par la présence de liaison entre les fibres et/ou les filaments. Cette consolidation peut être directe, c'est-à-dire faite directement entre les fibres et/ou filaments par soudure, ou elle peut être indirecte, c'est-à-dire par l'intermédiaire d'une couche intermédiaire entre les fibres et/ou les filaments, par exemple une couche de colle ou une couche de liant. Le terme non-tissé se rapporte à une structure en forme de ruban ou nappe de fibres et/ou filaments qui sont entrelacés d'une manière non uniforme, irrégulière ou au hasard. Un non-tissé peut avoir une structure de couche unique ou une structure à couches multiples. Un non-tissé peut également être réuni à un autre matériau pour former un stratifié. Un non-tissé peut être réalisé à partir de différents matériaux synthétiques et/ou naturels. Les matériaux naturels à titre d'exemple sont des fibres de cellulose, telles que le coton, la jute, le lin et analogue et peuvent également inclure des fibres de cellulose re-traitées, telles que la rayonne ou la viscose. Les fibres naturelles pour un matériau non-tissé peuvent être préparées en utilisant divers procédés tels que le cardage. Des matériaux synthétiques à titre d'exemple comportent, mais sans s'y limiter, des polymères plastiques synthétiques, qui sont connus pour former des fibres qui incluent, sans s'y limiter, les polyoléfines, par exemple le polyéthylène, polypropylène, polybutylène et analogue; le polyamide, par exemple le polyamide 6, polyamide 6.6, polyamide 10, polyamide 12 et analogue; des polyesters, par exemple des polyéthylènes téraphthalates, des polybutylènes téréphtalates, des acides polylactiques et analogues, des polycarbonates, des polystyrènes, des élastomères thermoplastiques, des vinyles polymères, des polyuréthanes et des mélanges et des copolymères de ces derniers. A titre d'exemple, le non-tissé peut être un **non-tissé** du type Spunbond, Spunmelt, cardé thermolié, SMS, SMMS, SS, SSS, SSMMS, SSMMMS, Air through ou autre. A titre d'exemple, le non-tissé peut être un non-tissé comprenant une combinaison différente de couches de Spunbond « S » et de Meltblown « M ». Ces exemples sont donnés à titre non limitatif.

La bande n'est pas limitée à un non-tissé, et peut plus généralement être un matériau tissé, un matériau tricoté, ou une combinaison de plusieurs de ces matériaux.

On entend par film thermoplastique un film en matériau thermoplastique qui peut être un matériau élastique ou un matériau non-élastique.

On entend par film thermoplastique en matériau élastique, un film qui peut être étire sans se rompre sous l'effet d'une force d'étirement exercée dans la direction latérale et pouvant reprendre sensiblement sa forme et ses dimensions initiales après relâchement de cette force d'étirement. Il s'agit par exemple d'un film qui conserve une déformation résiduelle ou rémanence après élongation et relâchement (déformation résiduelle aussi appelée « permanent set » ou « SET ») inférieure à 20%, plus préférentiellement inférieure à 5%, de sa dimension initiale (avant élongation) pour un allongement de 100% de sa dimension initiale, à température ambiante (23°C).

On entend par film thermoplastique en matériau non-élastique un film qui n'entre pas dans la définition d'un film thermoplastique en matériau élastique.

Dans certains modes de réalisation, la base est surmoulée sur la bande.

Dans certains modes de réalisation, la base a une épaisseur, mesurée perpendiculairement à la face supérieure de la base, supérieure ou égale à 10 $\mu$m, de préférence supérieure ou égale à 50 $\mu$m et inférieure ou égale à 700 $\mu$m, de préférence inférieure ou égale à 500 $\mu$m, encore plus de préférence inférieure ou égale à 100 $\mu$m.

Dans certains modes de réalisation, la base peut présenter une épaisseur constante ou non constante entre deux bords opposés de la base, par exemple entre deux bords opposés s'étendant dans la direction CD ou MD. La base peut être continue ou discontinue entre deux bords opposés de la base, par exemple entre deux bords opposés s'étendant dans la direction CD ou MD.

Dans certains modes de réalisation, la base a une épaisseur, mesurée perpendiculairement à la face supérieure de la base, comprise entre 10 et 700 $\mu$m et une hauteur de éléments de retenue, mesurée perpendiculairement à la face supérieure de la base, comprise entre 3 et 10 fois l'épaisseur de la base.

Dans certains modes de réalisation, la hauteur des éléments de retenue est supérieure ou égale à 35 $\mu$m, de préférence supérieure ou égale à 55 $\mu$m, encore plus de préférence supérieure ou égale à 80 $\mu$m et inférieure ou égale à 500 $\mu$m, de préférence inférieure ou égale à 350 $\mu$m, encore plus de préférence inférieure ou égale à 120 $\mu$m.

Par exemple, la hauteur des éléments de retenue peut être comprise entre 80 et 350 $\mu$m ou entre 55 et 120 $\mu$m.

Dans certains modes de réalisation, le diamètre dans lequel s'inscrit l'élément de retenue (en vue de dessus, perpendiculairement à l'élément de retenue) est supérieur ou égal à 80 $\mu$m, de préférence supérieur ou égal à 250 $\mu$m et inférieur ou égal à 500 $\mu$m, de préférence inférieur ou égal à 450 $\mu$m.

Dans certains modes de réalisation, une distance minimale entre deux éléments de retenue est comprise entre 0,1 mm et 10 mm.

Dans certains modes de réalisation, le motif présente une densité d'éléments de retenue supérieure ou égale à 20 éléments de retenue par cm$^2$, de préférence supérieure ou égale à 50 éléments de retenue par cm$^2$, encore plus de préférence supérieure ou égale à 100 éléments de retenue par cm$^2$ et inférieure ou égale à 250 éléments de retenue par cm$^2$, de préférence inférieure ou égale à 200 éléments de retenue par cm$^2$, encore plus de préférence inférieure ou égale à 150 éléments de retenue par cm$^2$.

Grâce à la force de pelage qui présente au moins deux pics consécutifs séparés par une vallée, la valeur des pics de la force de pelage allant croissante avec la course d'ouverture, l'utilisateur ressent cette force croissante requise pour séparer le dispositif de retenue de la zone d'application. Il perçoit donc que le dispositif de retenue était bien maintenu sur la zone d'application. Par ailleurs, la valeur maximale de la force de pelage est telle que le dispositif de retenue ne peut pas se détacher de la zone d'application de manière non désirée. Cela permet donc d'améliorer la sensation/qualité réelle et/ou perçue par utilisateur lorsque qu'il utilise le dispositif de retenue en fermeture et/ou en ouverture.

Les pics et les vallées ont une pointe et une base, la base peut présenter une largeur de préférence supérieure à 1 mm, plus particulièrement une largeur supérieure à 2 mm, dans certains cas, une largeur supérieure à 3 mm.

On considère généralement qu'une force de pelage, obtenue selon la méthode « Pelage 180° », qui est supérieure ou égale à 1,8 N permet d'éviter un détachement inopiné du dispositif de retenue et de la zone d'application et ce, indépendamment du dispositif de retenue et de la zone d'application.

Dans certains modes de réalisation, le motif est répétitif selon une direction de pelage, la force de pelage mesurée selon la méthode « Pelage 180° » présente au moins trois pics et au moins deux vallées, chaque vallée étant comprise entre deux pics consécutifs, les valeurs maximales des pics étant croissantes avec la course d'ouverture et les vallées présentant une valeur minimale inférieure ou égale à 85% d'une valeur maximale de la force de pelage, de préférence inférieure ou égale à 70%, encore plus de préférence inférieure ou égale à 60%, encore plus préférentiellement inférieure ou égale à 50%, encore plus préférentiellement inférieure ou égale à 40%.

Dans certains modes de réalisation, les zones dépourvues d'éléments de retenue qui sont continues selon une direction perpendiculaire à la direction de pelage présentent une largeur mesurée dans la direction de pelage supérieure ou égale à 2 rangées d'éléments de retenue mesurées dans la direction de pelage, de préférence supérieure ou égale à 3 rangées d'éléments de retenue mesurées dans la direction de pelage.

Dans certains modes de réalisation, les zones dépourvues d'éléments de retenue qui sont continues selon une direction perpendiculaire à la direction de pelage présentent une largeur mesurée dans la direction de pelage supérieure ou égale à 1% de la largeur de la base mesurée dans la direction de pelage, de préférence supérieure ou égale à 2%, de préférence supérieure ou égale à 4%, encore plus de préférence supérieure ou égale à 5%, encore plus de préférence supérieure ou égale à 10%.

Dans certains modes de réalisation, il existe plusieurs zones pourvues d'éléments de retenue, chaque zone pourvue d'éléments de retenue présentant, dans l'espace de couleur CIE L*a*b*, une différence de couleur $\Delta$E* entre la au moins une zone dépourvue d'éléments de retenue et au moins une partie du motif formé par la pluralité d'éléments de retenue, la différence de couleur $\Delta$E* étant supérieure ou égale à 1,0, de préférence supérieure ou égale à 1,5, encore plus de préférence supérieure ou égale à 3,0 encore plus de préférence supérieure ou égale à 4,5.

Dans certains modes de réalisation, il existe plusieurs zones pourvues d'éléments de retenue, chaque zone pourvue d'éléments de retenue présentant, dans l'espace de couleur CIE L*a*b*, une différence de couleur $\Delta$E* entre une première zone pourvue d'éléments de retenue et une deuxième zone pourvue d'éléments de retenue, la différence de couleur $\Delta$E* étant

supérieure ou égale à 1,0, de préférence supérieure ou égale à 1,5, encore plus de préférence supérieure ou égale à 3,0 encore plus de préférence supérieure ou égale à 4,5.

Dans certains modes de réalisation, l'élément de retenue comprend la tige surmontée d'une tête.

Dans certains modes de réalisation, une surface des zones dépourvues d'éléments de retenue est supérieure ou égale à 5% d'une surface totale de la base, de préférence est supérieure ou égale à 10%, encore plus de préférence est supérieure ou égale à 15%.

Dans certains modes de réalisation, le motif est un motif unique.

On entend par motif unique un motif isolé dans une zone de la base mesurant 25,4 mm ou 30,0 mm ou 35,0 mm dans la direction MD et 13,0 mm ou 25,0 mm ou 25,4 mm dans la direction CD.

Ce motif peut par exemple représenter un motif reconnaissable par l'utilisateur, par exemple un signe distinctif ou une marque.

Dans certains modes de réalisation, le motif est un motif répétitif.

Dans certains modes de réalisation, le motif répété peut être un motif complexe.

Dans certains modes de réalisation, le motif présente un contour fermé.

Dans certains modes de réalisation, le motif est agencé de sorte que lorsque le dispositif de retenue est sollicité en ouverture, une force exercée sur le dispositif de retenue est décomposée entre au moins une première composante en force de pelage et une deuxième composante en force de cisaillement.

Dans certains modes de réalisation, dans un espace de couleur CIE XYZ, la différence d'opacité entre les zones dépourvues d'éléments de retenue et au moins une partie du motif formé par la pluralité d'éléments de retenue est supérieure ou égale à 1, de préférence supérieure ou égale à 2, encore plus de préférence supérieure ou égale à 3.

Dans certains modes de réalisation, il existe plusieurs zones pourvues d'éléments de retenue, chaque zone pourvue d'éléments de retenue présentant, dans l'espace de couleur CIE XYZ, une différence d'opacité entre la au moins une zone dépourvue d'éléments de retenue et au moins une partie du motif formé par la pluralité d'éléments de retenue, la différence d'opacité étant supérieure ou égale à 1, de préférence supérieure ou égale à 2, encore plus de préférence supérieure ou égale à 3.

Dans certains modes de réalisation, il existe plusieurs zones pourvues d'éléments de retenue, chaque zone pourvue d'éléments de retenue présentant, dans l'espace de couleur CIE XYZ, une différence d'opacité entre une première zone pourvue d'éléments de retenue et une deuxième zone pourvue d'éléments de retenue, la différence d'opacité étant supérieure ou égale à 1, de préférence supérieure ou égale à 2, encore plus de préférence supérieure ou égale à 3.

Dans certains modes de réalisation, la base et la pluralité d'éléments de retenue sont en matériau thermoplastique.

Dans certains modes de réalisation, la base et la pluralité d'éléments de retenue sont d'un seul tenant.

En d'autres termes, la base et la pluralité d'éléments de retenue sont fait d'une même matière et il n'existe pas dans cette matière une interface à la jonction entre la base et la pluralité d'éléments de retenue.

Dans certains modes de réalisation, la base et la pluralité d'éléments de retenue sont en matériau thermoplastique comprenant un colorant.

Dans certains modes de réalisation, la base et la pluralité d'éléments de retenue sont en matériau thermoplastique comprenant au plus 2% massique de colorant, de préférence au plus 1,5% massique, encore plus de préférence au plus 1% massique de colorant.

Dans certains modes de réalisation, une extrémité de chaque élément de retenue la plus éloignée de la base comporte un revêtement coloré.

Dans certains modes de réalisation, le dispositif de retenue comprend une bande de tissé ou de non-tissé ou un film thermoplastique ou un film élastique ou un film composite.

Dans certains modes de réalisation, la base est surmoulée sur la bande.

Dans certains modes de réalisation, la base a une épaisseur, mesurée perpendiculairement à la face supérieure de la base, supérieure ou égale à 10 $\mu$m, de préférence supérieure ou égale à 50 $\mu$m et inférieure ou égale à 700 $\mu$m, de préférence inférieure ou égale à 500 $\mu$m, encore plus de préférence inférieure ou égale à 100 $\mu$m.

Dans certains modes de réalisation, la base peut présenter une épaisseur constante ou non constante entre deux bords opposés de la base, par exemple entre deux bords opposés s'étendant dans la direction CD ou MD. La base peut être continue ou discontinue entre deux bords opposés de la base, par exemple entre deux bords opposés s'étendant dans la direction CD ou MD.

Une hauteur des éléments de retenue, mesurée perpendiculairement à la face supérieure de la base est comprise entre 3 et 10 fois l'épaisseur de la base.

Dans certains modes de réalisation, la base a une épaisseur, mesurée perpendiculairement à la face supérieure de la base, comprise entre 10 et 700 $\mu$m et une hauteur de éléments de retenue, mesurée perpendiculairement à la face supérieure de la base, comprise entre 3 et 10 fois l'épaisseur de la base.

Dans certains modes de réalisation, la hauteur des éléments de retenue est supérieure ou égale à 35 $\mu$m, de préférence supérieure ou égale à 55 $\mu$m, encore plus de préférence supérieure ou égale à 80 $\mu$m et inférieure ou égale à 500 $\mu$m, de préférence inférieure ou égale à 350 $\mu$m, encore plus de préférence inférieure ou égale à 120 $\mu$m.

Par exemple, la hauteur des éléments de retenue peut être comprise entre 80 et 350 μm ou entre 55 et 120 μm.

Dans certains modes de réalisation, le diamètre dans lequel s'inscrit l'élément de retenue (en vue de dessus, perpendiculairement à l'élément de retenue) est supérieur ou égal à 80 μm, de préférence supérieur ou égal à 250 μm et inférieur ou égal à 500 μm, de préférence inférieur ou égal à 450 μm.

Dans certains modes de réalisation, une distance minimale entre deux éléments de retenue est comprise entre 0,1 mm et 10 mm.

Dans certains modes de réalisation, le motif présente une densité d'éléments de retenue supérieure ou égale à 20 par $cm^2$, de préférence supérieure ou égale à 50 par $cm^2$, encore plus de préférence supérieure ou égale à 100 par $cm^2$ et inférieure ou égale à 250 par $cm^2$, de préférence inférieure ou égale à 200 par $cm^2$, encore plus de préférence inférieure ou égale à 150 par $cm^2$.

Brève description des dessins

D'autres caractéristiques et avantages de l'objet du présent exposé ressortiront de la description suivante de modes de réalisation, donnés à titre d'exemples non limitatifs, en référence aux figures annexées, sur lesquelles :

- les figures 1A-1O sont des représentations schématiques de dispositifs de retenue selon le présent exposé présentant différents motifs ;
- la figure 2 est une vue en coupe de la figure 1A selon le plan de coupe II-II ;
- la figure 3 est une représentation schématique d'un exemple d'appareillage pour la réalisation d'un dispositif de retenue ;
- les figures 4 et 5 sont des représentations schématiques d'un exemple d'appareillage pour la réalisation d'un dispositif de retenue comprenant une bande de tissé ou de non-tissé ;
- la figure 6 est une vue de dessus en représentation schématique d'une partie de ruban pour dispositif de retenue ;
- la figure 7 est une vue de dessus en représentation schématique d'un autre ruban pour dispositif de retenue ;
- la figure 8 est une vue partielle agrandie du motif de la figure 1C ;
- la figure 9 est une vue d'un dispositif de retenue comprenant un revêtement coloré ;
- les figures 10 à 14 sont des graphiques représentant la force de pelage exprimée en newton en fonction de la course d'ouverture exprimée en millimètres respectivement pour les motifs 1K-1O ;
- les figures 15 et 16 sont des représentations schématiques d'équipements utilisés pour réaliser la mesure de la force de pelage.

Sur l'ensemble des figures, les éléments en commun sont repérés par des références numériques identiques.

Description détaillée

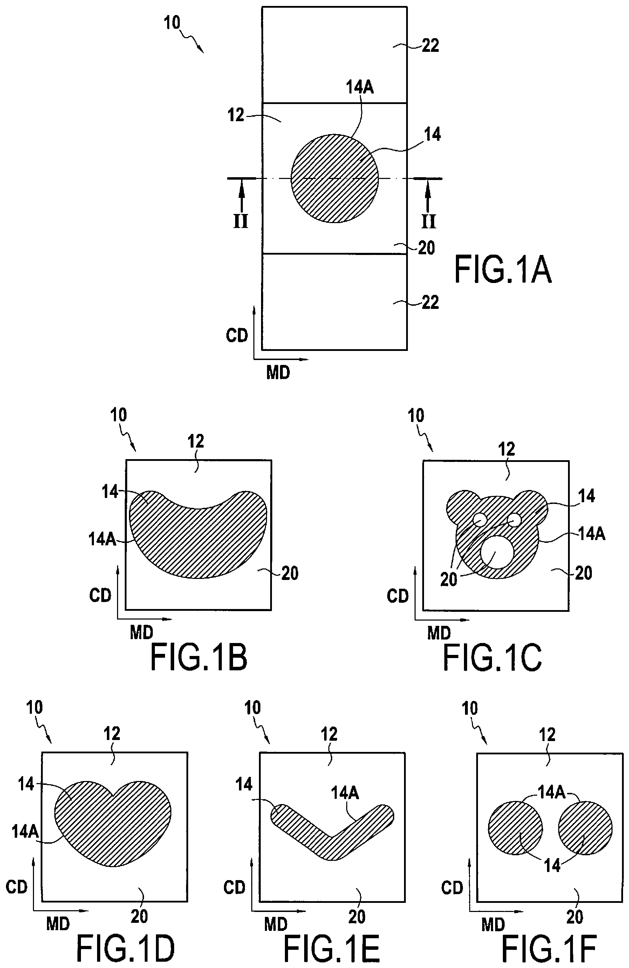

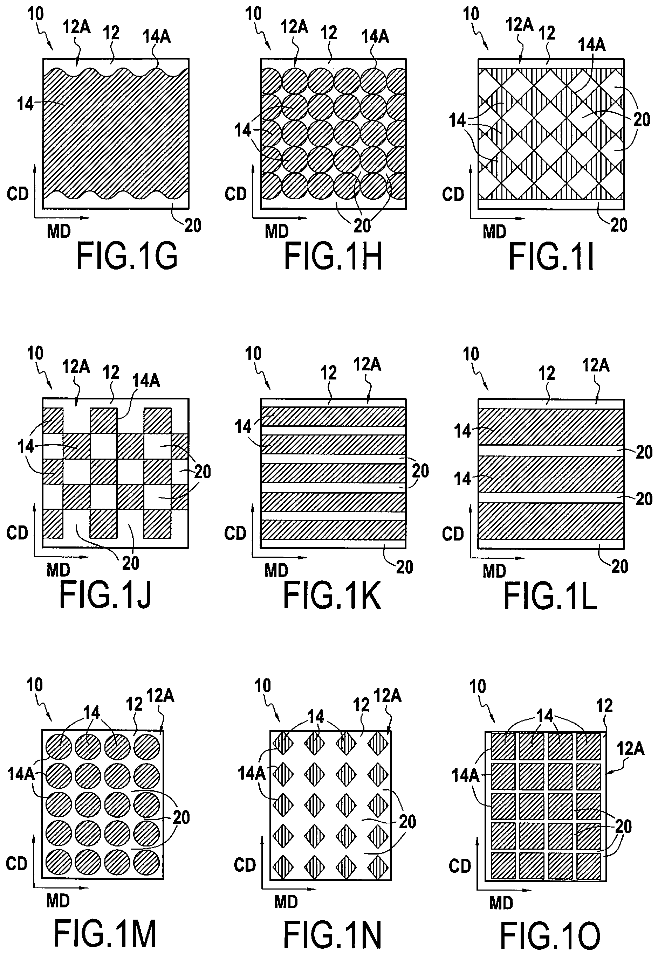

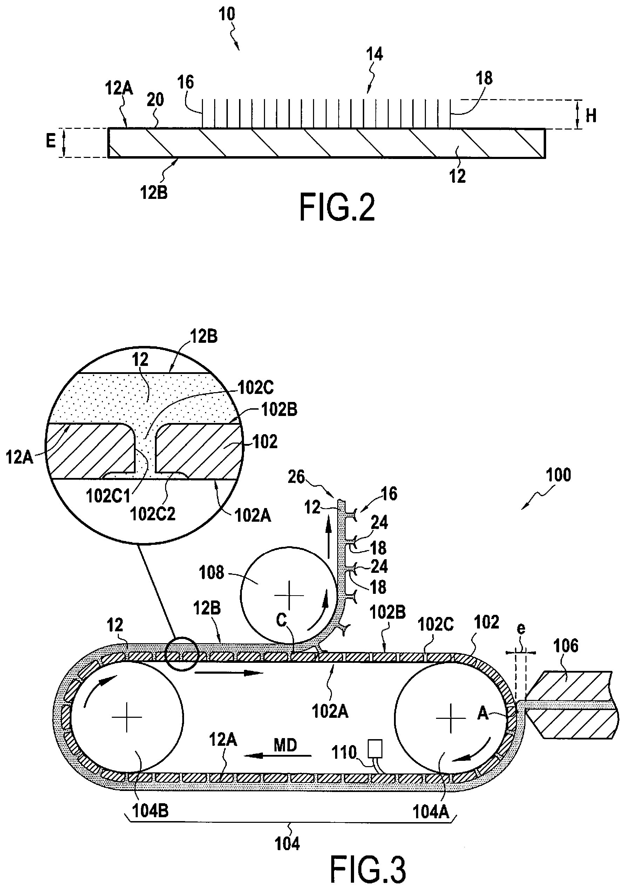

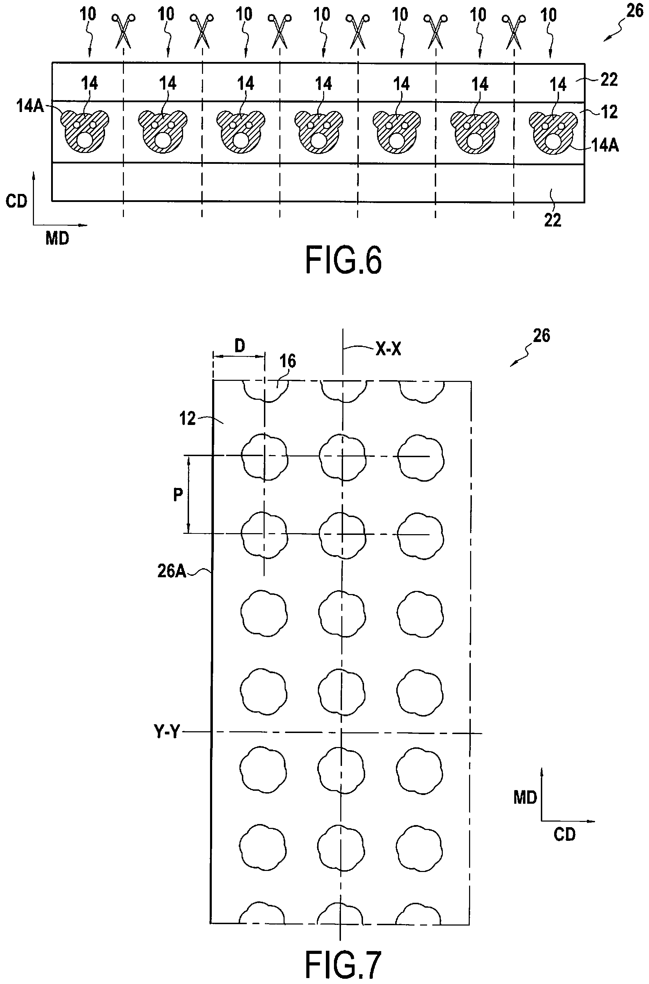

La figure 1A représente schématiquement un dispositif de retenue 10 comprenant une base 12 continue et un motif 14 en forme de disque plein. Comme représenté sur la figure 2, la base 12 comporte une face supérieure 12A et une face inférieure 12B et le motif 14 est formé par une pluralité d'éléments de retenue 16 s'étendant depuis la face supérieure 12A de la base 12. Chaque élément de retenue 16 comprend une tige 18. La base 12, en particulier la face supérieure 12A de la base 12, comprend également des zones 20 dépourvues d'éléments de retenue 16. Pour des raisons de simplification, les éléments de retenue 16 sont représentés par des hachures sur les figures 1A à 1O et 6.

Dans le mode de réalisation de la figure 1A, le dispositif de retenue 10 comprend une bande 22 de non-tissé (ou de tissé). Par exemple, la base 12 peut être surmoulée sur la bande 22 de non-tissé. La base 12 peut être également collée sur la bande 22 de non-tissé.

Dans ce qui suit, les éléments communs aux différents modes de réalisation sont identifiés par les mêmes références numériques.

De même, les figures 1B-1O présentent d'autres modes de réalisation du dispositif de retenue 10, en particulier du motif 14 formé par les éléments de retenue 16.

Comme on peut le voir sur les figures 1A-1B, le motif 14 peut être un motif unique (figures 1A-1E et 1G) ou un motif 14 répétitif (figures 1F et 1H-1O). Le motif 14 peut comprendre un contour fermé 14A (figures 1A-1F, 1H-1J et 1M-1O).

On notera que sur la figure 1G, les zones 20 dépourvues d'éléments de retenue 16 sont présentent sur deux bords du dispositif de retenue. Cependant, ces zones 20 présentent un bord ondulé qui définit le motif 14.

Comme représenté sur la figure 3, les éléments de retenue 16 peuvent comprendre la tige 18 surmontée d'une tête 24. La tige 18 comprend une extrémité inférieure reliée à la base 12, et une extrémité supérieure opposée à l'extrémité inférieure et la tête 24 surmonte l'extrémité supérieure de la tige 18. La base 12 et la pluralité d'éléments de retenue 16 sont d'un seul tenant. On comprend donc que la base 12 et la pluralité d'éléments de retenue 16 sont fait d'une même matière et qu'il n'existe pas dans cette matière une interface à la jonction entre la base et la pluralité d'éléments de retenue. Ce type d'élément de retenue 16 est généralement désigné par le terme de crochet ou préforme qui peut être ensuite modifiée, par exemple par calandrage, pour obtenir un crochet final. Un exemple de procédé de modification de la préforme est décrit dans le document FR3050620 (incorporée par référence).

Le dispositif de retenue 10 peut être fabriqué par exemple au moyen d'un appareillage 100 tel que représenté sur la figure 3. L'appareillage 100 permet de fabriquer un ruban 26 pour dispositif de retenue, le ruban 26 peut ensuite être découpé en une pluralité de dispositifs de retenue 10. Le ruban 26 comprend la base 12 continue et une pluralité d'éléments de retenue 16. Dans le mode de réalisation de la figure 3, les éléments de retenue 16 sont des crochets, chaque crochet comprenant une tige 18 surmontée d'une tête 24.

L'appareillage 100 tel que représenté comprend une bande de moulage 102 positionnée sur des moyens d'entrainement en rotation 104 comprenant ici deux rouleaux 104A, 104B, un moyen de distribution de matière 106, par exemple un injecteur, adapté pour réaliser une injection de matière de moulage par exemple thermoplastique et/ou élastique.

L'ensemble formé par la bande de moulage 102 et les moyens d'entrainement en rotation 104 forme ainsi un dispositif de moulage.

L'exemple illustré comprenant deux rouleaux 104A, 104B n'est pas limitatif, le nombre et l'agencement du ou des rouleaux peuvent varier notamment afin de s'adapter à la longueur de la bande de moulage 102 et aux différents postes de l'appareillage. On pourrait par exemple utiliser trois rouleaux ou encore un seul de telle sorte que la bande de moulage est agencée sur la périphérie du seul rouleau pour former un manchon ou « sleeve ». En particulier, un seul des deux rouleaux peut être entrainé en rotation par des moyens motorisés, par exemple le rouleau 104A, l'autre rouleau 104B étant libre, c'est à dire sans moyens motorisés, et entrainé en rotation via la bande de moulage, elle-même entrainée par le rouleau 104A.

La bande de moulage 102 telle que présentée comprend une face interne 102A et une face externe 102B, la face interne 102A étant au contact des moyens d'entrainement en rotation 104.

Le moyen de distribution de matière 106 est disposé de manière à injecter de la matière de moulage sur la face externe 102B de la bande de moulage 102.

Plus précisément, le moyen de distribution de matière 106 est disposé en regard de la bande de moulage 102, espacé de la bande de moulage 102 de manière à définir un entrefer e indiqué sur la figure 3. On repère par la référence A la limite de la matière injectée sur la face externe 102B de la bande de moulage 102, correspondant au front arrière de la matière injectée sur la bande de moulage 102 par rapport au sens de déplacement de la bande de moulage 102.

La bande de moulage 102 est munie d'une pluralité de cavités 102C permettant la réalisation de crochets du dispositif de retenue à crochets.

Les cavités 102C sont chacune formées de manière à définir une tige 102C1 s'étendant depuis la face externe 102B vers la face interne 102A de la bande de moulage 102 et une tête 102C2 s'étendant entre la tige 102C1 et la face interne 102A de la bande de moulage 102.

Dans l'exemple illustré, les têtes 24 des cavités 102C débouchent sur la face interne 102A de la bande de moulage 102. Les cavités 102C sont donc traversantes. Un tel mode de réalisation n'est pas limitatif, les cavités 102C peuvent également être borgnes, et donc ne pas déboucher de la face interne 102A de la bande de moulage 102 et/ou les cavités 102C peuvent ne comporter qu'une tige 102C1.

Les portions des cavités 102C formant les tiges 102C1 s'étendent typiquement selon une direction perpendiculaire à la face externe 102B de la bande de moulage 102. Les portions des cavités 102C formant les tiges 102C1 ont typiquement une géométrie de rotation autour d'un axe perpendiculaire à la face externe 102B de la bande de moulage 102, ou une géométrie présentant un plan de symétrie s'étendant selon une direction parallèle au sens de défilement de la bande de moulage 102 et/ou selon une direction perpendiculaire au sens de défilement de la bande de moulage 102.

Les portions des cavités 102C formant les têtes 102C2 s'étendent typiquement radialement ou transversalement par rapport à un axe perpendiculaire à la face externe 102B de la bande de moulage 102, et peuvent présenter une symétrie de rotation autour de cet axe perpendiculaire à la face externe 102B de la bande de moulage 102. Les portions des cavités 102C formant les têtes 102C2 présentent typiquement une forme sensiblement tronconique ou hexaédrique.

Les portions des cavités 102C formant les têtes 102C2 peuvent être linéaires ou incurvées, par exemple pour former des portions incurvées vers la face interne 102A ou vers la face externe 102B de la bande de moulage 102 s'étendant depuis les portions des cavités 102C formant les tiges 102C1.

Les portions des cavités 102C formant les têtes 102C2 peuvent présenter une épaisseur constante ou variable.

Dans l'exemple représenté sur les figures, les portions des cavités 102C formant les têtes 102C2 s'étendent radialement autour des portions des cavités 102C formant les tiges 102C1, et présentent une forme générale de disque.

La bande de moulage 102 peut présenter sur sa face interne 102A ou sur sa face externe 102B une texturation particulière telle que des rainures, réseau de gorges ou réseau de passage formant évent ou picots, ou être sensiblement lisse.

La bande de moulage 102 peut être formée par une superposition de plusieurs bandes, et n'est donc pas nécessairement monobloc ou monomatière.

Le moyen de distribution de matière 106 est typiquement disposé de manière à réaliser l'injection de matériau de moulage dans la bande de moulage 102 en une section de la bande de moulage 102 où cette dernière est en appui contre un rouleau d'entrainement, en l'occurrence le rouleau d'entrainement 104A dans l'exemple représenté sur la figure 3. Le rouleau d'entrainement forme alors un fond pour les cavités 102C.

Dans le cas où l'injection de matériau de moulage est réalisée alors que la bande de moulage 102 n'est pas en appui contre un rouleau d'entrainement, le moyen de distribution de matière 106 peut alors comprendre une base disposée de l'autre côté de la bande de moulage 102, de sorte que la face interne 102A de la bande de moulage 102 soit en appui contre la base lorsque l'injection de matière est réalisée, la base formant alors un fond pour les cavités 102C de la bande de moulage 102.

L'utilisation d'une bande de moulage 102 associée à des moyens d'entrainement 104 par rapport à l'utilisation de moyens de formation conventionnels tels que des rouleaux dans lesquels sont directement réalisées des cavités de moulage est avantageuse pour plusieurs raisons.

L'utilisation d'une bande de moulage 102 est notamment intéressante en termes de modularité. La bande de moulage peut en effet être retirée et remplacée facilement des moyens d'entrainement, contrairement à un rouleau massif pour lequel les opérations de démontage et remontage sont particulièrement complexes à réaliser. Un tel avantage s'observe particulièrement lorsque les deux rouleaux 104A, 104B sont fixés à un bâti d'un seul et même côté, laissant l'extrémité de l'autre côté libre pour introduire/retirer la bande de moulage. Un moyen de guidage de la bande de moulage peut également être utilisé afin d'en faciliter l'introduction et/ou le retrait.

De plus, la réalisation d'une bande de moulage est fortement simplifiée par rapport à la réalisation d'un rouleau comprenant des cavités de moulage. De tels rouleaux sont en effet typiquement réalisés par empilement de tranches successives, nécessitant donc de multiples opérations d'usinage et entrainant des contraintes importantes lors de l'assemblage et à chaque changement de référence de crochets et présente une masse importante nécessitant le maintien de ces rouleaux par leurs deux extrémités, ce qui complexifie par conséquent leur remplacement.

Les cavités 102C dans la bande de moulage 102 peuvent être réalisées par un procédé d'attaque chimique ou par utilisation d'un laser aux endroits où l'on souhaite former des éléments de retenue 16. On peut également envisager de réaliser la bande de moulage 102 avec des cavités 102C réparties uniformément sur toute la bande de moulage 102 et de venir ensuite boucher les cavités 102C aux endroits où l'on souhaite former des zones 20 dépourvues d'éléments de retenue 16.

On repère sur la figure 3 par la référence C la séparation entre le ruban 26 et la bande de moulage 102, ce point correspondant par exemple au niveau à partir duquel la base 12 du ruban 26 n'est plus au contact de la bande de moulage 102. On pourra prévoir que la bande de moulage 102 embarre sur le rouleau de démoulage 108, c'est-à-dire que le rouleau de démoulage 106 forme un levier dans la bande de moulage 102 pour faciliter le démoulage des préformes et/ou crochets.

Dans l'exemple représenté, les cavités 102C de la bande de moulage 102 sont traversantes. L'appareillage peut alors comprendre un élément, tel qu'une racle 110, positionné de manière à racler la face interne 102A de la bande de moulage 102 pour retirer au besoin le matériau de moulage excédentaire. On entend par injection, l'action de mise en forme d'une matière de moulage par voie fondue, par exemple, la distribution, l'apport, le moulage, l'injection, l'extrusion.

L'appareillage présenté précédemment et le procédé associé peuvent également présenter des moyens et une étape d'association d'une bande 22 de non-tissé (ou de tissé) à la base 12.

Une telle association d'une bande 22 sur une base 12 comprenant des éléments de retenue 16 est typiquement réalisée au moyen d'un adhésif, ou via une fusion de la base ou de la bande.

Afin de réaliser une telle solidarisation d'une bande 22, par exemple en non-tissé, à la base 12 du dispositif de retenue 10, l'appareillage 100 proposé peut comprendre des moyens d'entrainement de bande 22, adaptés pour réaliser une alimentation en bande et pour appliquer la bande contre la face inférieure 12B de la base 12 en aval du moyen de distribution de matière 106.

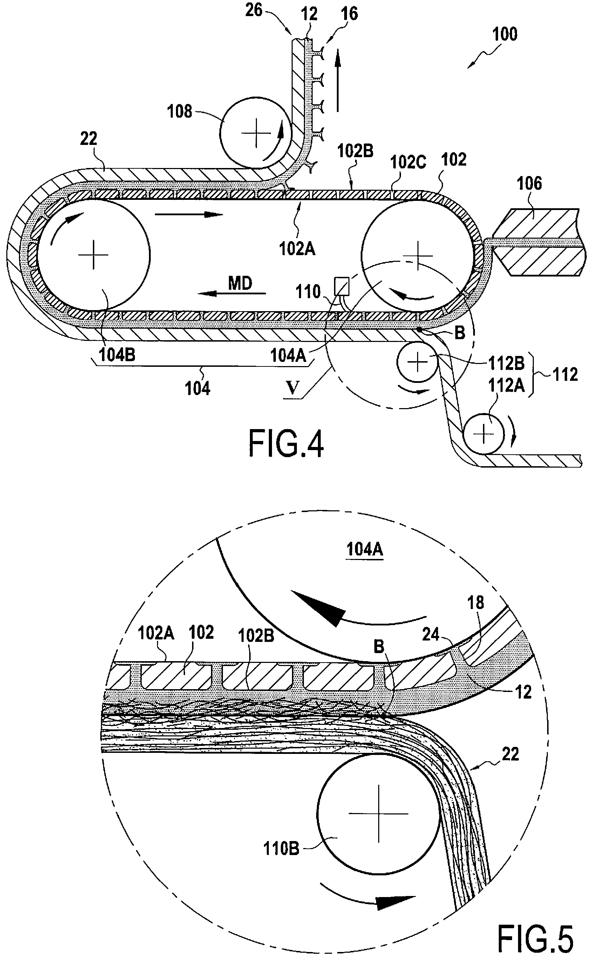

On représente schématiquement sur les figures 4 et 5 un exemple d'appareillage 100 comprenant de tels moyens.

L'appareillage tel qu'illustré est similaire à celui présenté précédemment en référence à la figure 3 ; les éléments en commun ne sont donc pas décrits à nouveau ici.

Comme on le voit sur les figures 4 et 5, l'appareillage tel que présenté comprend des moyens d'entrainement de bande 112, ici constitués de deux rouleaux 112A, 112B, configurés pour réaliser une alimentation en bande 22 en aval du moyen de distribution de matière 106.

La bande 22 est typiquement une couche de matériau non-tissé, un film thermoplastique, un film élastique ou un film composite, ou encore un ensemble de fibres et/ou filaments consolidé thermiquement. La bande 22 est par exemple une nappe de fibres et/ou filaments.

Dans l'exemple représenté sur les figures 4 et 5, la bande est représentée comme étant une couche de matériau non-tissé.

Les moyens d'entrainement de substrat 110 sont configurés pour alimenter l'appareillage en bande 22, et appliquer cette bande 22 contre la face inférieure 12B de la base 12 en aval du moyen de distribution de matière 106.

Les moyens d'entrainement de substrat 110 sont configurés de manière à ce que cette application soit réalisée préalablement à la solidification de la base 12. Ainsi, cette application entraine une pénétration au moins partielle de la bande 22 au-delà d'un plan défini par la face inférieure 12B de la base 12. On repère par la référence B sur les figures le point de mise en contact entre la base 12 et la bande 22.

Plus précisément, la face inférieure 12B de la base 12 est sensiblement plane, et définit un plan. L'application du substrat contre cette face entraine une pénétration de portions de la bande 22, par exemple de fibres et/ou filaments de la couche de matériau non-tissé dans le cas où la bande 22 est une couche de matériau non-tissé au sein de la base 12, traversant de ce fait la face inférieure 12B de la base 12.

Dans la mesure où une telle application est réalisée préalablement à la solidification de la base 12, il n'est pas nécessaire de chauffer la base 12 et/ou la bande 22 afin de réaliser une telle liaison.

A titre d'exemple, en considérant une base 12 réalisée en polypropylène, l'application du substrat contre la face inférieure 12B de la base 12 est typiquement réalisée lorsque la face inférieure 12B de la base 12 présente une température comprise entre la température de fusion du matériau et la température de ramollissement Vicat B du matériau la constituant moins 30°C (degré Celsius) ou encore entre la température de fusion du matériau la constituant et la température de ramollissement Vicat A du matériau la constituant. Plus particulièrement, lorsque la base comprend un matériau à base de polypropylène, la face inférieure 12B de la base 12 présente une température comprise entre 75°C et 150°C, typiquement de l'ordre de 105°C, cette température étant typiquement mesurée au moyen d'une caméra infrarouge ou laser. On entend par température de ramollissement VICAT la température obtenue selon l'une des méthodes décrites dans les normes ISO 306 ou ASTM D 1525 avec une vitesse de chauffe de 50°C/h et une charge normalisée de 50N pour le VICAT B et une charge normalisée de 10N pour le VICAT A.

La bande 22 peut être appliquée de manière uniforme ou non uniforme contre la face inférieure 12B de la base 12.

La liaison réalisée entre la bande 22 et la base 12 peut être réalisée de manière uniforme ou non uniforme.

Dans le cas où la bande 22 est un ensemble de fibres et/ou filaments consolidés thermiquement, la liaison avec la base 12 est également réalisée par pénétration dans la base 12 d'une partie des fibres et/ou filaments de la bande 22.

Dans le cas où la bande 22 est un ensemble de fibres et/ou filaments consolidés thermiquement, un film thermoplastique, un film élastique ou un film composite, il peut résulter alors de la liaison avec la base un phénomène de retassure de la base 12 lors de son refroidissement, cette retassure favorisant la surface de liaison entre le substrat et la base du ruban. Cette retassure est sans impact sur l'aspect visuel pour l'utilisateur final.

Dans le cas où la bande 22 est une couche de matériau non-tissé, le démoulage des crochets est réalisé de manière aisée même avec un non-tissé dont le grammage est inférieur à 80 g/m$^2$ (masse de matière en gramme par mètre carré de non-tissé). A titre d'exemple, le grammage du non-tissé peut être compris entre 5 g/m$^2$ et 120 g/m$^2$, ou encore entre 25 g/m$^2$ et 100 g/m$^2$, ou encore entre 10 g/m$^2$ et 70 g/m$^2$.

Dans le cas où la bande 22 est une couche de matériau non-tissé, l'appareillage peut comprendre un dispositif de calandrage en amont des moyens d'entrainement de substrat 112, permettant ainsi de réaliser une étape de calandrage localement ou non de la couche de matériau non-tissé préalablement à son application contre la base 12.

Ce mode de solidarisation d'une bande 12 à une base 12 comprenant des éléments de retenue 16 est notamment avantageux en ce qu'il n'entraine pas une déformation de la base 12, et permet donc avantageusement de conserver la forme de la base 12 obtenue lors de l'étape d'injection, et notamment de conserver les bords droits pouvant être obtenus via le procédé et l'appareillage décrits précédemment.

Ce mode de solidarisation d'un substrat à un ruban peut être appliqué à un procédé de formation d'un ruban tel que décrit précédemment, ou plus généralement à tout autre procédé de formation d'un ruban comprenant des éléments de retenue tels que des crochets.

La figure 6 représente schématiquement une partie de ruban 26 obtenu au moyen de l'appareillage 100 de la figure 4.

Le ruban 26 est destiné à être découpé en une pluralité de dispositifs de retenue 10. L'emplacement des découpes est représenté par les traits en pointillés sur la figure 6. Le ruban 26 et chaque dispositif de retenue 10 comprend une base 12 continue en matière thermoplastique et des éléments de retenue 16 formant des motifs 14. Dans ce mode de réalisation, les motifs 14 sont les motifs 14 de la figure 1C.

La figure 7 représente un ruban 26 en vue de dessus, lequel ruban 26 comprend une base 12 et des éléments de retenues 16, ici des préformes ou crochets. Par simplicité, les éléments de retenue 16 ont été agrandi et sont représentés uniformément répartis sur la base 12.

La figure 7 représente schématiquement le ruban 26 obtenu avec l'appareillage 100 de la figure 3. Le ruban 26 s'étendant donc selon une direction longitudinale repérée par un axe X-X sur la figure 7, qui est parallèle à la direction MD. On représente également sur la figure 7 la direction transverse, repérée par un axe Y-Y, qui est parallèle à la direction CD.

On définit pour ce ruban 26 un bord 26As'étendant selon la direction MD, ce bord 26A définissant une des deux extrémités du ruban 26 selon la direction CD.

Les éléments de retenue 16, comprenant une tige 8 surmontée ou non d'une tête 24, sont généralement agencés à proximité du bord 26A. Les éléments de retenue 16 sont typiquement agencés à une distance D du bord 26A comprise entre 2 et 3 pas P entre les éléments de retenue, typiquement égale à 2 ou 3 pas P des éléments de retenue, la distance D étant mesurée selon la direction transversale par rapport à la direction longitudinale matérialisée par l'axe X-X sur la figure 7. Le pas P entre deux éléments de retenue 16 correspond à la distance entre le centre de deux éléments de retenue successifs selon la direction longitudinale. Dans l'exemple représenté sur la figure 7, les éléments de retenue 16 sont agencés en colonnes s'étendant selon la direction longitudinale matérialisée par l'axe X-X, ces colonnes étant répétées à l'identique selon la direction transversale.

On notera que la figure 7 n'est pas une représentation à l'échelle. En effet, la distance D entre un bord 26A et le premier élément de retenue 16 est représentée comme étant inférieure au rayon R du cercle 28 correspondant au pas moyen. Le centre de chaque cercle est positionné, en vue du dessus, sur le centre de chaque élément de retenue et la circonférence d'un premier cercle passe par le centre d'un élément de retenue adjacent. Le pas moyen peut correspondre à la distance séparant deux éléments de retenue adjacents, c'est-à-dire le rayon R du cercle 28. Or, la distance D est typiquement égale à 2 ou 3 pas moyens. Le diamètre (égal à deux fois le rayon R) dans lequel s'inscrit l'élément de retenue (en vue de dessus, perpendiculairement à l'élément de retenue) est supérieur ou égal à 80 $\mu$m, de préférence supérieur ou égal à 250 $\mu$m et inférieur ou égal à 500 $\mu$m, de préférence inférieur ou égal à 450 $\mu$m.

On notera que sur le côté gauche de la figure 7, le dispositif de retenue 10 ne comporte pas de zone dépourvue d'élément de retenue 16 au sens du présent exposé. En effet, la distance D défini une bande rectiligne le long du bord 26A de la base 12 dépourvue d'éléments de retenue. Toutefois, cette bande ne permet pas de définir un motif au sens du présent exposé, en ce que cette bande n'est pas bordée de chaque côté, dans la direction perpendiculaire au bord, par des dispositifs de retenue.

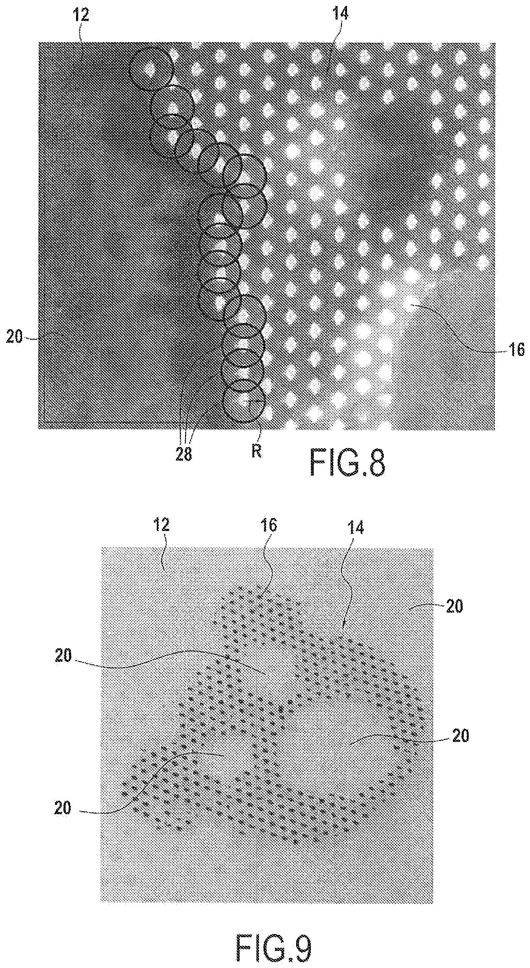

Les éléments de retenue 16 peuvent également être agencés en quinconce ou « nid d'abeille », par exemple en décalant les colonnes d'éléments de retenue selon la direction longitudinale, comme par exemple cela est représenté sur la figure 8.

Sur la figure 8, seuls quelques cercles ont été représentés du côté gauche de la figure.

On comprend que la bande de moulage 102 présente des cavités 102C disposées de manière à former le motif 14 de la figure 8.

On comprend que ce type de dispositif de retenue 10 peut être obtenu par d'autres appareillages que des appareillages à bande de moulage 102. Cependant, on comprend que la réalisation d'une bande de moulage 102 comprenant des cavités 102C réparties de sorte que les éléments de retenue 16 forment un motif 14 sur la base 12 est plus facile que la réalisation d'un rouleau, par exemple. En outre, il est relativement aisé de changer la bande de moulage 102 de l'appareillage 100 des figures 3 à 5 lorsque l'on souhaite réaliser un motif 14 différent.

A titre d'exemple non limitatif, la matière injectée par le moyen de distribution de matière 106 pour former la base 12 et les éléments de retenue 16 peut comprendre une matière thermoplastique incolore ou une matière thermoplastique avec un colorant. La matière thermoplastique peut notamment être du polypropylène.

Le colorant peut être par exemple un colorant blanc, par exemple on peut citer la référence 50PP commercialisée par CABOT et qui est chargé à 50% massique de $TiO_2$. Comme autre colorant, on peut également citer un colorant violet de référence UN55206 fabriqué par COLOR SERVICE. Ces exemples sont donnés à titre non limitatif.

Dans tous les exemples cités, la densité des éléments de retenue 16 dans le ou les motifs 14, est égale à 280 éléments de retenue 16 par $cm^2$ de base 12, les éléments de retenue 16 présentant un pas (distance de centre à centre de deux éléments de retenue adjacents) entre deux éléments de retenue égale à 0,64 mm. Cette densité d'éléments de retenue est donnée à titre d'exemple non limitatif. La base 12 présente une épaisseur E, mesurée perpendiculairement à la face supérieure 12A de la base 12, égale à 60 $\mu$m (voir figure 2). Les éléments de retenue 16 présentent une hauteur H, mesurée perpendiculairement à la base 12, qui est égale à 5 fois l'épaisseur de la base 12.

La hauteur H des éléments de retenue peut être supérieure ou égale à 35 $\mu$m, de préférence supérieure ou égale à 55 $\mu$m, encore plus de préférence supérieure ou égale à 80 $\mu$m et inférieure ou égale à 500 $\mu$m, de préférence inférieure ou égale à 350 $\mu$m, encore plus de préférence inférieure ou égale à 120 $\mu$m.

Par exemple, la hauteur H des éléments de retenue peut être comprise entre 80 et 350 $\mu$m ou entre 55 et 120 $\mu$m.

Le diamètre dans lequel s'inscrit l'élément de retenue (en vue de dessus, perpendiculairement à l'élément de retenue) est supérieur ou égal à 80 $\mu$m, de préférence supérieur ou égal à 250 $\mu$m et inférieur ou égal à 500 $\mu$m, de préférence inférieur ou égal à 450 $\mu$m.

Exemple 1 : on peut injecter un mélange de polypropylène et de colorant blanc de référence 50PP commercialisée par CABOT et qui est chargé à 50% massique de $TiO_2$. Le mélange injecté peut comprendre 99,2 % massique de polypropylène et 0,8 % massique de colorant blanc.

Exemple 2 : on peut injecter un mélange de polypropylène, de colorant violet de référence UN55206 fabriqué par COLOR SERVICE et de colorant blanc de référence 50PP commercialisée par CABOT et qui est chargé à 50% massique de $TiO_2$. Le mélange injecté peut comprendre 99,2 % massique de polypropylène, 0,4 % massique de colorant blanc et 0,4 % massique de colorant violet.

Exemple 3 : l'exemple 3 présente la même composition que l'exemple 1 et comporte un revêtement coloré qui est déposé sur les éléments de retenue 16 en utilisant une encre et/ou un colorant, tels que ceux couramment utilisés en flexographie et/ou en tampographie, par exemple une encre noire, comme représenté sur la figure 9.

Exemple 4 : l'exemple 4 présente la même composition que l'exemple 2 et comporte un revêtement coloré qui est déposé sur les éléments de retenue 16 en utilisant une encre et/ou un colorant, tels que ceux couramment utilisés en flexographie et/ou en tampographie, par exemple une encre noire, comme représenté sur la figure 9.

Dans l'espace CIE L*a*b*, les paramètres L*, a* et b* sont mesurés respectivement pour des zones 20 dépourvues d'éléments de retenue et des parties du motif 14 formé par les éléments de retenue 16. Les valeurs de L*, a* et b* sont mesurées par exemple avec un spectrocolorimètre en lumière naturelle (sous la référence D65/10°) de modèle RM200QC de x-rite pantone sur un support blanc et la différence de couleur ∆E* entre les zones 20 dépourvues d'éléments de retenue et au moins une partie du motif 14 formé par la pluralité d'éléments de retenue 16 est calculée selon l'équation (1).

Pour l'exemple 1, la différence de couleur ∆E* est égale à 1,606 ; pour l'exemple 2, la différence de couleur ∆E* est égale à 5,493 ; pour l'exemple 3, la différence de couleur ∆E* est égale à 7,352 et pour l'exemple 4, la différence de couleur ∆E* est égale à 7,911.

On comprend que la différence de couleur est présente sur les dispositifs de retenue dès lors que la base 12 comprend des zones 20 dépourvues d'éléments de retenue 16. Ainsi, la différence de couleur entre les zones 20 dépourvues d'éléments de retenue et au moins une partie du motif 14 formé par la pluralité d'éléments de retenue 16 permet d'améliorer la sensation/qualité réelle et/ou perçue par utilisateur lorsque qu'un utilisateur utilise le dispositif de retenue en fermeture et/ou en ouverture. On comprend que cet avantage peut être obtenu de manière indépendante de la forme du motif 14.

Dans l'espace de couleur CIE XYZ, la différence d'opacité entre les zones 20 dépourvues d'éléments de retenue et au moins une partie du motif 14 formé par la pluralité d'éléments de retenue 16 est mesurée par exemple avec un spectrocolorimètre en lumière naturelle (sous la référence D65/10°) de modèle RM200QC de x-rite pantone. Les valeurs de $Y_{fond\ noir}$ et $Y_{fond\ blanc}$ sont mesurées sur un support blanc et sur un support noir respectivement pour les zones 20 dépourvues d'éléments de retenue et au moins une partie du motif 14 formé par la pluralité d'éléments de retenue 16 et l'opacité exprimée en % est calculée selon l'équation (2) respectivement pour les zones 20 dépourvues d'éléments de retenue et au moins une partie du motif 14 formé par la pluralité d'éléments de retenue 16.

La différence d'opacité entre les zones 20 dépourvues d'éléments de retenue et au moins une partie du motif 14 formé par la pluralité d'éléments de retenue 16 est égale à la valeur absolue de la différence des valeurs d'opacité obtenues pour la zone 20 dépourvue dépourvues d'éléments de retenue et pour au moins une partie du motif 14 formé par la pluralité d'éléments de retenue 16.

Pour l'exemple 1, la différence d'opacité est égale à 0,45 ; pour l'exemple 2, la différence d'opacité est égale à 3,67 ; pour l'exemple 3, la différence d'opacité est égale à 1,87 et pour l'exemple 4, la différence d'opacité est égale à 12,00.

Comme on peut le constater, la différence d'opacité permet de renforcer la perception de la différence de couleur ∆E* perçue par l'utilisateur. En effet, pour l'exemple 1, la différence de couleur ∆E* étant supérieure à 1, l'utilisateur peut voir une différence entre les zones 20 dépourvues d'éléments de retenue et le motif 14. Cependant, la différenciation entre les zones 20 dépourvues d'éléments de retenue et le motif 14 est meilleure pour l'exemple 2 pour lequel la différence de couleur ∆E* est égale à 5,493 et la différence d'opacité est égale à 3,67.

Les motifs 14 des figures 1A-1O sont généralement utilisé selon la direction CD, c'est-à-dire que le pelage, c'est-à-dire la séparation du dispositif de retenue 10 d'une zone d'application à boucle par exemple se fait parallèlement à la direction CD.

Les motifs 14 des figures 1A-1D pourraient aussi être pivotés de 90°.

Le motif 14 de la figure 1E est préférentiellement utilisé comme représenté sur cette figure. En effet, la forme du motif 14 étant sensiblement un « V », la séparation du dispositif de retenue 10 d'une zone d'application transforme la force appliqué selon la direction CD pour détacher ou séparer le dispositif de retenue 10 d'une zone d'application en une composante en force de pelage et en une composante en force de cisaillement. On comprend que le motif 14 de la figure 1E est agencé de sorte que lorsque le dispositif de retenue 10 est sollicité en ouverture, une force exercée sur le dispositif de retenue 10 est décomposée entre au moins une première composante en force de pelage et une deuxième composante en force de cisaillement. Il en va de même du motif de la figure 1F.

On notera que les motifs 14 des figures 1K à 1O présentent, dans la direction CD, une alternance de zones 20 dépourvues d'éléments de retenue 16 et de motifs 14 et également, dans la direction MD, des zones 20 dépourvues d'éléments de retenue 16 continues.

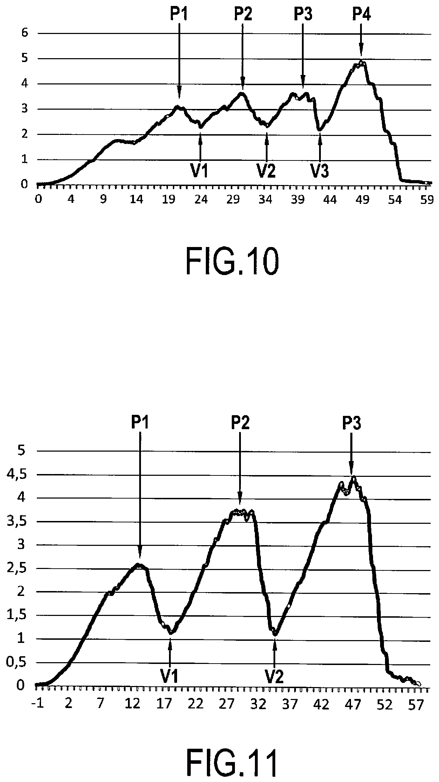

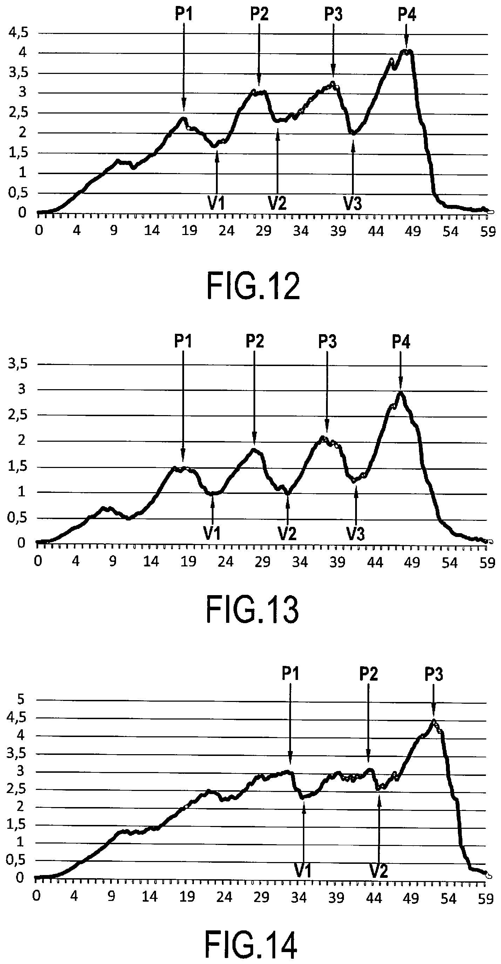

Pour les motifs 1K à 1O, on a représenté respectivement sur les figures 10 à 14 les courbes de pelage obtenues avec la méthode « Pelage 180° » en utilisant une zone d'application 30 commercialisée par APLIX sous la référence « SoftLoop Premium by APLIX », comprenant un non-tissé à boucle du type cardé thermolié et un non-tissé SMS assemblé par calandrage à chaud.

Les courbes de pelages présentent la force de pelage exprimée en N (ordonnée) en fonction de la course d'ouverture exprimée en millimètres (abscisse). Les courbes présentées aux figures 10 à 14 sont des moyennes réalisées sur 20 mesures de différents échantillons. La direction de pelage est parallèle à la direction CD pour tous les dispositifs de retenue testés.

Les dispositifs de retenue 10 selon les motifs des figures 1K à 1O sont préparés conformément à la méthode « Pelage 180° » décrite précédemment.

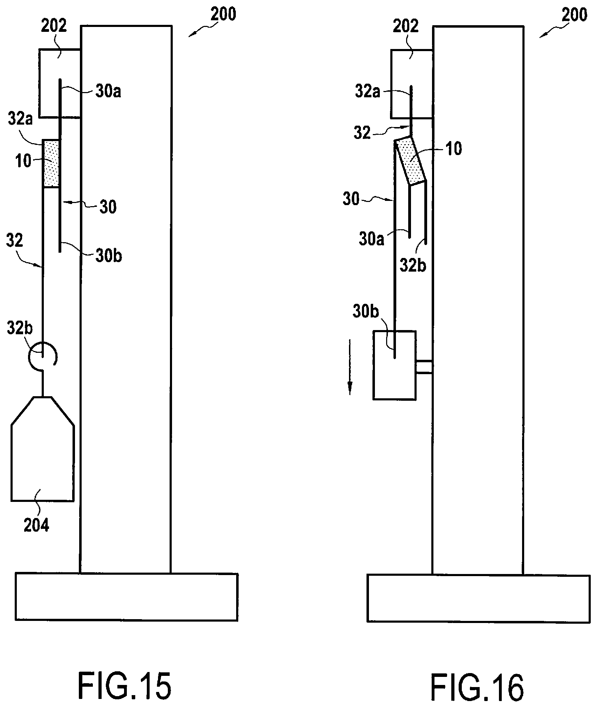

La figure 15 représente une bandelette 32 comprenant le dispositif de retenue 10 à tester, le dispositif de retenue étant assemblé sur la zone d'application 30. L'échantillon de la zone d'application 30 est disposé dans une pince 202 d'une potence 200, le côté coupé 30a étant dans la pince 202 et un poids 204 de 1 kg est suspendu à la partie inférieure 32b de la bandelette 32 pendant 10 s (seconde). Le poids 204 est ensuite retiré.

L'ensemble est ensuite disposé dans une machine d'essai en traction 210 comprenant une cellule de mesure de 100 N. L'extrémité 32a de la bandelette 32, c'est-à-dire l'extrémité opposée à la partie inférieure 32b, est insérée dans la mâchoire supérieure 212. On met la lecture de la cellule de mesure de force à zéro. On insère l'extrémité coupée 30a de l'échantillon de la zone d'application 30, c'est-à-dire le côté opposé au côté non-coupé 30b) dans la mâchoire inférieure 214 et on crée une légère tension. La force doit être comprise entre 0,02 N et 0,05 N. Lors de la mise en place, les mâchoires 212, 214 sont écartées l'une de l'autre de 50 mm. L'ensemble est centré entre les deux mâchoires 212, 214. Le test est réalisé à déplacement constant à une vitesse de 305 mm/min (millimètre par minute) et la course d'essai est de 50 mm. Cette course d'essai est ajustée en fonction de la largeur du dispositif de retenue à tester de manière à permettre la désolidarisation totale du dispositif de retenue et de la zone d'application.

Comme on peut le constater sur les figures 10 à 14, les courbes de pelage présentent des pics successifs P1-P4, chaque pic présentant une valeur maximale supérieure à la valeur maximale du pic qui le précède dans la sens de la course d'ouverture, c'est-à-dire qu'entre deux pics consécutifs pris deux à deux, la valeur maximale du pic présentant l'abscisse la plus grande est supérieure à la valeur maximale du pic présentant l'abscisse la plus petite, et inversement, la valeur maximale du pic présentant l'abscisse la plus petite est inférieure à la valeur maximale du pic présentant l'abscisse la plus grande. Ainsi, sur la figure 10, la valeur maximale du pic P1 est inférieure à la valeur maximale du pic P2 ; la valeur maximale du pic P2 est inférieure à la valeur maximale du pic P3 ; la valeur maximale du pic P3 est inférieure à la valeur maximale du pic P4. On notera également que la valeur minimale des vallées V1-V3 est inférieure à 50% à une valeur maximale de la force de pelage, c'est-à-dire à la valeur maximale du pic P4.

Il en va de même pour les motifs des figures 1L-1N.

Les pics et les vallées ont une pointe et une base, la base peut présenter une largeur de préférence supérieure à 1 mm, plus particulièrement une largeur supérieure à 2 mm, dans certains cas, une largeur supérieure à 3 mm.

Grâce à la force de pelage qui présente au moins deux pics consécutifs séparés par une vallée, la valeur des pics de la force de pelage allant croissante avec la course d'ouverture, l'utilisateur ressent cette force croissante requise pour séparer le dispositif de retenue de la zone d'application. Il perçoit donc que le dispositif de retenue était bien maintenu sur la zone d'application. Par ailleurs, la valeur maximale de la force de pelage est telle que le dispositif de retenue ne peut pas se détacher de la zone d'application de manière non désirée.

On constate que pour le motif de la figure 1O, la vallée V2 présente une valeur minimale qui est supérieure à 50% de la valeur maximale du pic P3. On constate également que les différents pics P1-P3 ne sont pas marqués de manière aussi identifiable que sur les autres courbes (figures 10 à 13). Cela est lié notamment à la largeur L mesurée selon la direction CD des zones 20 dépourvues d'éléments de retenue 16 continues selon la direction MD.

On comprend que les courbes de pelage dépendent également de la nature de zone d'application 30. Toutefois, des zones d'application différentes de la zone d'application commercialisée par APLIX sous la référence « SoftLoop Premium by APLIX » donneront des courbes de pelage présentant un profil similaire, bien que les valeurs de la force de pelage puissent varier en fonction de la zone d'application.

On comprend que les courbes de pelage sont indépendantes de la couleur et/ou de la différence de couleur $\Delta E^*$ entre les zones 20 dépourvues d'éléments de retenue et au moins une partie du motif 14 formé par la pluralité d'éléments de retenue 16.

Cependant, ces deux caractéristiques peuvent être combinées pour renforcer la sensation/qualité réelle et/ou perçue par utilisateur lorsque qu'un utilisateur utilise le dispositif de retenue en fermeture et/ou en ouverture.

Quoique le présent exposé ait été décrit en se référant à un exemple de réalisation spécifique, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif.

Le présent exposé peut trouver une application dans le domaine de l'hygiène, des couches culottes, de l'incontinence adulte, de l'industrie de l'emballage, par exemple de l'emballage alimentaire, de la stomie, du bâtiments et autre.

**Revendications**

1. Dispositif de retenue (10) destiné à coopérer avec une contrepartie à crochets et/ou à boucles comprenant :

- une base (12) continue présentant une face supérieure (12A) et une face inférieure (12B), la base (12) ayant une épaisseur, mesurée perpendiculairement à la face supérieure de la base ; et
- une pluralité d'éléments de retenue (16) s'étendant de la face supérieure (12A) de la base (12), les éléments de retenue étant des tiges et/ou des préformes et/ou des crochets, chaque élément de retenue (16) comprenant une tige (18), une hauteur des éléments de retenue, mesurée perpendiculairement à la face supérieure (12A) de la base est comprise entre 3 et 10 fois l'épaisseur de la base ;

la base (12) comportant au moins une zone (20) continue selon une direction perpendiculaire à une direction de pelage, la zone (20) étant dépourvue d'éléments de retenue de sorte que la pluralité d'éléments de retenue (16) forme au moins deux motifs (14) et dans lequel le motif (14) est répétitif selon la direction de pelage, la base (12) comportant une alternance de zones (20) dépourvues d'éléments de retenue (16) et de motifs (14), la force de pelage mesurée selon la méthode « Pelage 180° » présentant au moins deux pics (P1-P4) et au moins une vallée (V1-V3) comprise entre les deux pics (P1-P4), des valeurs maximales des pics étant croissantes avec la course d'ouverture et la au moins une vallée présentant une valeur minimale inférieure ou égale à 85% d'une valeur maximale de la force de pelage, de préférence inférieure ou égale à 70%, encore plus de préférence inférieure ou égale à 60%, encore plus préférentiellement inférieure ou égale à 50%, encore plus préférentiellement inférieure ou égale à 40%, une distance minimale entre deux éléments de retenue (16) étant comprise entre 0,1 mm et 10 mm, et le motif (14) présentant une densité d'éléments de retenue (16) supérieure ou égale à 20 éléments de retenue (16) par cm$^2$.

2. Dispositif de retenue (10) selon la revendication 1, dans lequel la base (12) comporte au moins deux zones (20) continues selon une direction perpendiculaire à la direction de pelage dépourvue d'éléments de retenue de sorte que la pluralité d'éléments de retenue (16) forme au moins trois motifs (14) et dans lequel le motif (14) est répétitif selon la direction de pelage, la force de pelage mesurée selon la méthode « Pelage 180° » présente au moins trois pics et au moins deux vallées, chaque vallée étant comprise entre deux pics consécutifs, les valeurs maximales des pics étant croissantes avec la course d'ouverture et les vallées présentant une valeur minimale inférieure ou égale à 85% d'une valeur maximale de la force de pelage, de préférence inférieure ou égale à 70%, encore plus de préférence inférieure ou égale à 60%, encore plus préférentiellement inférieure ou égale à 50%, encore plus préférentiellement inférieure ou égale à 40%.

3. Dispositif de retenue (10) selon la revendication 1 ou 2, dans lequel les zones (20) dépourvues d'éléments de retenue qui sont continues selon une direction perpendiculaire à la direction de pelage présentent une largeur mesurée dans la direction de pelage supérieure ou égale à 2 rangées d'éléments de retenue mesurées dans la direction de pelage, de préférence supérieure ou égale à 3 rangées d'éléments de retenue mesurées dans la direction de pelage.

4. Dispositif de retenue (10) selon la revendication 1 ou 2, dans lequel les zones dépourvues d'éléments de retenue qui sont continues selon une direction perpendiculaire à la direction de pelage présentent une largeur mesurée dans la direction de pelage supérieure ou égale à 1% de la largeur de la base mesurée dans la direction de pelage, de préférence supérieure ou égale à 2%, de préférence supérieure ou égale à 4%, encore plus de préférence supérieure ou égale à 5%, encore plus de préférence supérieure ou égale à 10%.

5. Dispositif de retenue (10) selon l'une quelconque des revendications 1 à 4, dans lequel, dans un espace de couleur CIE L*a*b*, une différence de couleur ΔE* entre la au moins une zone dépourvue d'éléments de retenue et au moins une partie du motif formé par la pluralité d'éléments de retenue est supérieure ou égale à 1,0, de préférence supérieure ou égale à 1,5, encore plus de préférence supérieure ou égale à 3,0 encore plus de préférence supérieure ou égale à 4,5.

6. Dispositif de retenue (10) selon l'une quelconque des revendications 1 à 5, dans lequel l'élément de retenue comprend la tige surmontée d'une tête.

7. Dispositif de retenue (10) selon l'une quelconque des revendications 1 à 6, dans lequel une surface des zones dépourvues d'éléments de retenue est supérieure ou égale à 5% d'une surface totale de la base, de préférence est supérieure ou égale à 10%, encore plus de préférence est supérieure ou égale à 15%.

8. Dispositif de retenue (10) selon l'une quelconque des revendications 1 à 7, comprenant une bande (22) de tissé ou de non-tissé ou un film thermoplastique ou un film élastique ou un film composite.

9.  Dispositif de retenue (10) selon l'une quelconque des revendications 1 à 8, dans lequel la base (12) a une épaisseur (E), mesurée perpendiculairement à la face supérieure (12A) de la base (12), comprise entre 10 et 700 μm.

**Patentansprüche**

1.  Haltevorrichtung (10), die dazu bestimmt ist, mit einem Gegenstück mit Haken und/oder Schlaufen zusammenzuwirken, umfassend:

    - eine durchgehende Basis (12), die eine Oberseite (12A) und eine Unterseite (12B) aufweist, wobei die Basis (12) eine Stärke aufweist, die senkrecht zu der Oberseite der Basis gemessen wird; und
    - eine Vielzahl von Halteelementen (16), die sich von der Oberseite (12A) der Basis (12) erstrecken, wobei die Halteelemente Stäbe und/oder Vorformen und/oder Haken sind, jedes Halteelement (16) umfassend einen Schaft (18), wobei eine Höhe der Halteelemente, gemessen senkrecht zu der Oberseite (12A) der Basis, zwischen dem 3- und 10-fachen der Stärke der Basis liegt;

    die Basis (12) umfassend mindestens einen Bereich (20), der in einer Richtung senkrecht zu einer Schälrichtung durchgehend ist, wobei der Bereich (20) frei von Halteelementen ist, sodass die Vielzahl von Halteelementen (16) mindestens zwei Muster (14) bildet, und wobei sich das Muster (14) entlang der Schälrichtung wiederholt, die Basis (12) umfassend eine Abfolge von Bereichen (20), die frei von Halteelementen (16) und Mustern (14) sind, wobei die gemäß dem Verfahren "Schälwinkel 180°" gemessene Schälkraft mindestens zwei Spitzen (P1-P4) und mindestens ein zwischen den zwei Spitzen (P1-P4) liegendes Tal (V1-V3) aufweist, wobei die Maximalwerte der Spitzen mit dem Öffnungshub zunehmen und das mindestens eine Tal einen Minimalwert aufweist, der kleiner als oder gleich wie 85 % eines Maximalwerts der Schälkraft ist, vorzugsweise kleiner als oder gleich wie 70 %, bevorzugter kleiner als oder gleich wie 60 %, noch bevorzugter kleiner als oder gleich wie 50 %, noch bevorzugter kleiner als oder gleich wie 40 %, ein Mindestabstand zwischen zwei Halteelementen (16) zwischen 0,1 mm und 10 mm liegt, und wobei das Muster (14) eine Dichte an Halteelementen (16) von 20 oder mehr Halteelementen (16) pro cm$^2$ aufweist.

2.  Haltevorrichtung (10) nach Anspruch 1, wobei die Basis (12) mindestens zwei durchgehende Bereiche (20) in einer Richtung senkrecht zu der Schälrichtung umfasst, die frei von Halteelementen sind, sodass die Vielzahl von Halteelementen (16) mindestens drei Muster (14) bildet, und wobei sich das Muster (14) in der Schälrichtung wiederholt, wobei die gemäß dem Verfahren "Schälwinkel 180°" gemessene Schälkraft mindestens drei Spitzen und mindestens zwei Täler aufweist, wobei jedes Tal zwischen zwei aufeinanderfolgenden Spitzen liegt, wobei die Maximalwerte der Spitzen mit dem Öffnungshub ansteigen und die Täler einen Minimalwert aufweisen, der kleiner als oder gleich wie 85 % eines Maximalwerts der Schälkraft ist, vorzugsweise kleiner als oder gleich wie 70 %, bevorzugter kleiner als oder gleich wie 60 %, noch bevorzugter kleiner als oder gleich wie 50 %, noch bevorzugter kleiner als oder gleich wie 40 %.

3.  Haltevorrichtung (10) nach Anspruch 1 oder 2, wobei die Bereiche (20), die frei von Halteelementen sind, die in einer Richtung senkrecht zu der Schälrichtung durchgehend sind, eine in der Schälrichtung gemessene Breite aufweisen, die größer als oder gleich wie 2 in der Schälrichtung gemessenen Halteelementreihen ist, vorzugsweise größer als oder gleich wie 3 in der Schälrichtung gemessenen Halteelementreihen ist.

4.  Haltevorrichtung (10) nach Anspruch 1 oder 2, wobei die Bereiche, die frei von Halteelementen sind, die in einer Richtung senkrecht zu der Schälrichtung durchgehend sind, eine in der Schälrichtung gemessene Breite aufweisen, die größer als oder gleich wie 1 % der in der Schälrichtung gemessenen Breite der Basis ist, vorzugsweise größer als oder gleich wie 2 %, vorzugsweise größer als oder gleich wie 4 %, bevorzugter größer als oder gleich wie 5 %, bevorzugter größer als oder gleich wie 10 %.

5.  Haltevorrichtung (10) nach einem der Ansprüche 1 bis 4, wobei in einem CIE-Farbraum L*a*b* ein Farbunterschied ΔE* zwischen dem mindestens einen Bereich, der frei von Halteelementen ist, und mindestens einem Teil des durch die Vielzahl von Halteelementen gebildeten Musters größer als oder gleich wie 1,0, vorzugsweise größer als oder gleich wie 1,5, bevorzugter größer als oder gleich wie 3,0 bevorzugter größer als oder gleich wie 4,5 ist.

6.  Haltevorrichtung (10) nach einem der Ansprüche 1 bis 5, wobei das Halteelement den Stab umfasst, worauf ein Kopf montiert ist.

7.  Haltevorrichtung (10) nach einem der Ansprüche 1 bis 6, wobei eine Fläche der Bereiche, die frei von Halteelementen sind, größer als oder gleich wie 5 % einer Gesamtfläche der Basis ist, vorzugsweise größer als oder gleich wie 10 % ist, bevorzugter größer als oder gleich wie 15 % ist.

8. Haltevorrichtung (10) nach einem der Ansprüche 1 bis 7, umfassend ein Band (22) aus einem gewebten oder nicht gewebten Stoff oder einen thermoplastischen Film oder einen elastischen Film oder einen Verbundfilm.

9. Haltevorrichtung (10) nach einem der Ansprüche 1 bis 8, wobei die Basis (12) eine Stärk (E), gemessen senkrecht zu der Oberseite (12A) der Basis (12), zwischen 10 und 700 μm aufweist.

**Claims**

1. A retention device (10) intended to cooperate with a hook and/or loop counterpart comprising:

   - a continuous base (12) having an upper face (12A) and a lower face (12B), the base (12) having a thickness measured perpendicularly to the upper face of the base ; and
   - a plurality of retention elements (16) extending from the upper face (12A) of the base (12), the retention elements being rods and/or preforms and/or hooks, each retention element (16) comprising a rod (18), a height of retention elements, measured perpendicularly to the upper face (12A) of the base is comprised between 3 and 10 times the thickness of the base ;

   the base (12) including at least one zone (20) that is continuous in a direction perpendicular to the peeling direction, the zone (20) being free of retention elements so that the plurality of retention elements (16) form at least two patterns (14), and wherein the pattern (14) is repetitive in the peeling direction, the base (12) comprising an alternation of zones (20) free of retention elements (16) and of patterns (14), the peeling force measured according to the "180° peeling" method having at least two peaks (P1-P4) and at least one valley (V1-V3) comprised between the two peaks (P1-P4), maximum values of the peaks increasing as peeling progresses and the at least one valley having a minimum value less than or equal to 85% of a maximum value of the peeling force, preferably less than or equal to 70%, even more preferably less than or equal to 60%, even more preferably less than or equal to 50%, even more preferably less than or equal to 40%, a minimum distance between two retention elements (16) being comprised between 0.1 mm and 10 mm, and the pattern (14) having a density of retention elements (16) greater than or equal to 20 retention elements (16) per cm$^2$.

2. The retention device (10) according to claim 1, wherein the base (12) includes at least two zones (20) that are continuous in a direction perpendicular to the peeling direction free of retention elements so that the plurality of retention elements (16) form at least three patterns (14), and wherein the pattern (14) is repetitive in a peeling direction, the peeling force measured according to the "180° peeling" method has at least three peaks and at least two valleys, each valley being comprised between two consecutive peaks, the maximum values of the peaks increasing as peeling progresses and the valleys having a minimum value less than or equal to 85% of a maximum value of the peeling force, preferably less than or equal to 70%, even more preferably less than or equal to 60%, even more preferably less than or equal to 50%, even more preferably less than or equal to 40%.

3. The retention device (10) according to claim 1 or 2, wherein the zones (20) free of retention elements that are continuous in a direction perpendicular to the peeling direction have a width measured in the peeling direction greater than or equal to 2 rows of retention elements measured in the peeling direction, preferably greater than or equal to 3 rows of retention elements measured in the peeling direction.

4. The retention device (10) according to claim 1 or 2, wherein the zones free of retention elements that are continuous in a direction perpendicular to the peeling direction have a width measured in the peeling direction greater than or equal to 1% of the width of the base measured in the peeling direction, preferably greater than or equal to 2%, preferably greater than or equal to 4%, even more preferably greater than or equal to 5%, even more preferably greater than or equal to 10%.

5. The retention device (10) according to claims 1 to 4, wherein in a CIE L*a*b* color space, a color difference ΔE* between the at least one zone free of retention elements and at least part of the pattern formed by the plurality of retention elements is greater than or equal to 1.0, preferably greater than or equal to 1.5, even more preferably greater than or equal to 3.0, even more preferably greater than or equal to 4.5.

6. The retention device (10) according to claims 1 to 5, wherein the retention device includes the rod surmounted by a head.

7. The retention device (10) according to claims 1 to 6, wherein a surface of the zones free of retention elements is greater than or equal to 5% of a total surface of the base, preferably greater than or equal to 10%, even more preferably greater than or equal to 15%.

8. The retention device (10) according to claims 1 to 7, comprising a woven (22) or non-woven web or a thermoplastic film or an elastic film or a composite film.

9. The retention device (10) according to claims 1 to 8, wherein the base (12) has a thickness (E), measured perpendicularly to the upper face (12A) of the base (12), comprised between 10 and 700 μm.

FIG.1A

FIG.1B

FIG.1C

FIG.1D

FIG.1E

FIG.1F

FIG.1G

FIG.1H

FIG.1I

FIG.1J

FIG.1K

FIG.1L

FIG.1M

FIG.1N

FIG.1O

FIG.2

FIG.3

FIG.4

FIG.5

**FIG.6**

**FIG.7**

FIG.8

FIG.9

FIG.10

FIG.11

FIG.12

FIG.13

FIG.14

FIG.15

FIG.16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20140358107 A
- US 2016128877 A
- FR 3050620